# EUROPEAN PATENT SPECIFICATION

(11) **EP 3 337 413 B1**
(45) Date of publication and mention of the grant of the patent: **30.09.2020**
(21) Application number: 16784560.1
(22) Date of filing: 29.07.2016
(51) Int. Cl.: A61B 17/3205, A61B 17/34

(54) **EXTRACTOR FOR REMOVING A LEAD FROM A PATIENT**
EXTRAKTOR ZUM ENTFERNEN EINER ELEKTRODE VON EINEM PATIENTEN
EXTRACTEUR POUR RETIRER UNE SONDE DU CORPS D'UN PATIENT

(30) Priority: 17.08.2015 US 201514828353
(43) Date of publication of application: 27.06.2018
(73) Proprietor: Talpanetics B.V., 3751 DL Bunschoten-Spakenberg (NL)
(72) Inventor: KALMANN, Menno, 8075PD Elpeet (NL); KAPTEIN, Wieger, 3751 DL Bunschoten-Spakenberg (NL); DROR, Amit, Ben, 20104 Zurit (IL)
(74) Representative: Lees, Kate Jane
(86) International application number: PCT/IB2016/001187
(87) International publication number: WO 2017/029548

(56) References cited:
- WO-A1-2015/028882
- US-A1- 2011 106 099
- US-A1- 2013 116 704

## Description

### BACKGROUND

### Technical Field

The invention relates to an extractor for removing an implanted lead from a patient, such as a cardiac pacing lead.

### Background of Related Art

Recently, implantation of cardiac pacing devices has become a standard medical intervention for correcting cardiac rhythm thereby reducing patient's health complaints due to an abnormal cardiac condition.

The cardiac pacing device, such as a pacemaker, includes one or more electrical leads which supply a due electrical stimulus from the pacemaker or implantable cardioverter defibrillator to the heart muscle. These electrodes are implanted in the heart tissue, i.e., in a vein in the heart such as the superior vena cava or subclavia vena which may take place during open heart surgery. The distal portion of the electrical leads may include anchors for affixing the electrode lead inside the heart muscle. The electrode wire is covered with a suitable layer of insulator for electrical safety in operation. The leads can have an externally threaded tip to screw into the tissue.

During use, the electrical lead may be damaged or may need to be replaced due to maintenance considerations. This procedure is usually complicated by the fact that during the time the lead has dwelled inside the body, it has grown into a scar tissue as well as it may be covered by tissue as the result of tissue ingrowth. Tissue ingrowth can occur along various portions of the lead. It is appreciated that both phenomena make it difficult to remove the electrode lead from the heart tissue. This is especially the case since the vein makes a curve from the pacemaker to the heart and the lead is often attached to the vein at this curve, thereby making release difficult.

Through the years different attempts have been made to provide a suitable lead extractor device which is capable of removing an implanted electrical lead without causing damage to the patient.

Originally, lead extractors were mechanical devices operable by a cardiac surgeon to free the leads from the surrounding tissue for removing them from the heart. The disadvantage of such devices is that a mechanical force is initially applied in the region of a manifold of the lead extractor and has to be suitably transferred to a distant location along the lead for freeing it from the tissue. Usually the lead extraction is carried out using a subclavian approach or femoral approach. In both approaches a sheath is placed over the lead and is threaded over the lead to reach the distal portion, i.e., the tip, of the lead. However, it has been clinically found that such mechanical approach has a high risk of undesirable disruption of the tissue of the patient when attempting to free the implanted electrode lead from the heart muscle. Also, the hardened tissue around the lead can in some instances make placement of the sheath difficult.

A particular version of a lead extractor is disclosed in US Patent No. 4,574,800, which is arranged to remove implanted leads from a patient by grasping the lead substantially close to its implantation position. Accordingly, this extractor device includes an elongate tubular member arranged to slide into and through a longitudinal lumen of the cardiac pacing lead. The distal portion of the elongate tubular member comprises a protrusion member adapted to provide a wedging surface. The wedging surface is effected by a tapering proximal surface of the protrusion member. The proximal tapering surface may take the form of a spherical or a conical section. The elongate tubular member further includes a spherical gripping member arranged to engage with the lead. When the proximal end portion of the elongate member is pulled with a substantial force, for example, by suitable actuation of the handle, the protrusion member forms a flared distal end section of the elongate tubular member. The elongate tubular member has a length such that it projects beyond the proximal end of the cardiac lead when the known extractor is fully inserted into the lead. In use, the extractor assembly is inserted into and through the cardiac pacing lead until the protrusion member abuts the proximal end of the implanted electrode. Afterwards, the protrusion member is activated to cause the distal portion of the tubular member to wedge. The wedged portion comes into frictional engagement with the inside surface of the distal portion of the cardiac pacing lead. Finally, a pulling force is applied to the proximal portion of the elongate tubular member, which is transmitted to the distal portion of the elongate tubular member towards the flared portion. This pulls the cardiac pacing lead from its dwelling.

Although in the foregoing system's excessive force to the electrode wire and its insulator sheath may be avoided, the pulling forces, which are transferred from the proximal end of the lead extractor, may cause undesirable local damage to the tissue. Additionally, since the lead extractor is provided inside the lumen of the lead, it has to meet stringent constraints regarding its permissible dimensions. This limits the possibilities of optimization of the lead extractor in terms of mechanics.

Other prior art attempts to extract leads involve inserting a tube over the lead and drilling down with the tube to separate surrounding tissue from the external surface of the lead to free the lead. Still other prior art methods include utilizing lasers or electrosurgical energy, such as radiofrequency energy at the end of a catheter to sever the tissue.

WO2015/028882 describes an extractor comprising a proximal portion, a distal portion, a lumen dimensioned to receive an implanted lead therein, a cutter at the distal portion for cutting tissue adjacent the implanted lead, and a first clamping member movable between a clamping position to clamp the lead and an unclamping position to unclamp the lead.

The need exists for a simplified and less traumatic approach to removing leads, such as cardiac leads, from a patient.

### SUMMARY

The present device provides an improved lead extractor which is capable of secure removal of the implanted leads, such as cardiac leads, causing minimum damage to the patient's tissue. The lead is clamped by the extractor and incremental relative movement of the lead and retractor moves the lead within the extractor lumen as tissue surrounding the lead is cut (dissected) by the extractor.

According to the present invention there is provided an extractor for removing an implanted lead from a patient, the extractor comprising a lumen dimensioned to receive the lead therein; a cutter at a distal portion for cutting tissue adjacent the lead; a distal clamping structure spaced proximally of the cutter; and a proximal clamping structure spaced proximally of the distal clamping structure, the proximal clamping structure relatively movable with respect to the distal clamping structure to extract the lead from the patient, wherein the proximal clamping structure includes a first plurality of discrete radially spaced apart clamping members.

In some embodiments each of the clamping members has a series of teeth adapted to frictionally engage the lead to apply a sufficient gripping force to effect relative movement of the lead when it is pulled proximally.

In some embodiments the extractor further includes a movement mechanism operatively associated with the proximal clamping structure, the movement mechanism movable between first and second positions to move the proximal clamping structure between distal and proximal positions.

In some embodiments movement of the proximal clamping structure proximally effects extraction of the lead and movement of the proximal clamping structure distally resets the proximal clamping structure for subsequent movement of the proximal clamping structure for further extraction of the lead.

In some embodiments the proximal clamping structure comprises a first plurality of springs, the first plurality of springs biasing the first plurality of clamping members in a direction toward a longitudinal axis of the extractor.

In some embodiments the distal clamping structure is substantially stationary and enables relative movement of the lead in response to proximal movement of the proximal clamping structure but prevents relative movement of the lead in a reverse direction. The distal clamping structure may include a second plurality of discrete radially spaced apart clamping members. The extractor may further comprise a second plurality of springs, the second plurality of springs biasing the second plurality of clamping members in a direction toward a longitudinal axis of the extractor.

In some embodiments, the extractor further comprises a cable operatively associated with the proximal clamping structure, wherein proximal movement of the cable retracts the proximal clamping structure proximally to effect extraction of the lead. Distal movement of the cable may reset the proximal clamping structure for subsequent proximal movement to further extract the lead.

In some embodiments the cutter is rotatable concurrently with axial movement of the proximal clamping structure.

In some embodiments the extractor further comprises a flexible sheath, the flexible sheath rotatable with the extractor to unscrew a distal tip of the lead from tissue.

In some embodiments the extractor further comprises an override mechanism to release the proximal and distal clamping structures to enable removal of the extractor from the lead. The distal clamping structure may include a second plurality of radially spaced apart clamping members, and the override mechanism may include a slidable member engageable with the first and second plurality of clamping members to force the first and second plurality of clamping members out of frictional engagement with the lead against the force of a plurality of springs.

In some embodiments the extractor and lead are incrementally relatively movable to swallow the lead as tissue is cut by the cutter adjacent the lead.

### BRIEF DESCRIPTION OF THE DRAWINGS

Preferred embodiment(s) of the present disclosure are described herein with reference to the drawings wherein:
Figure 1 illustrates a patient's anatomy showing an implanted cardiac lead to be removed;
Figure 2 is a perspective view of one embodiment of a lead extractor not forming part of the present invention;
Figure 3 is a perspective view of the lead extractor of Figure 2 positioned over the cardiac lead;
Figure 4 is a side view of the lead extractor of Figure 2 with the outer housing shown in phantom to illustrate the internal rings and springs, the extractor shown in the neutral position;
Figure 5 is a side perspective view similar to Figure 4 with the housing removed for clarity;
Figure 6 is a side view of the lead extractor of Figure 2 showing the distal clamp ring moved to the angled position;
Figure 7 is a side view similar to Figure 6 showing the proximal clamp ring moved to the angled position;
Figure 8 is a close up view a portion of the cutter (knife) of the housing for cutting tissue;
Figure 9 is a close up view of the distal end of the housing of Figure 2;
Figure 10 is a front view illustrating the cable extending through the notch in the proximal clamp ring;
Figures 11A-11C are schematic views of an embodiment not forming part of the present invention utilizing a motor to move (oscillate) the cables of the lead extractor to alternatively angle and retract the distal and proximal clamping rings;
Figures 12A-12F illustrate side views (with the housing shown in phantom) to show the method of use of the lead extractor of Figure 2 wherein:

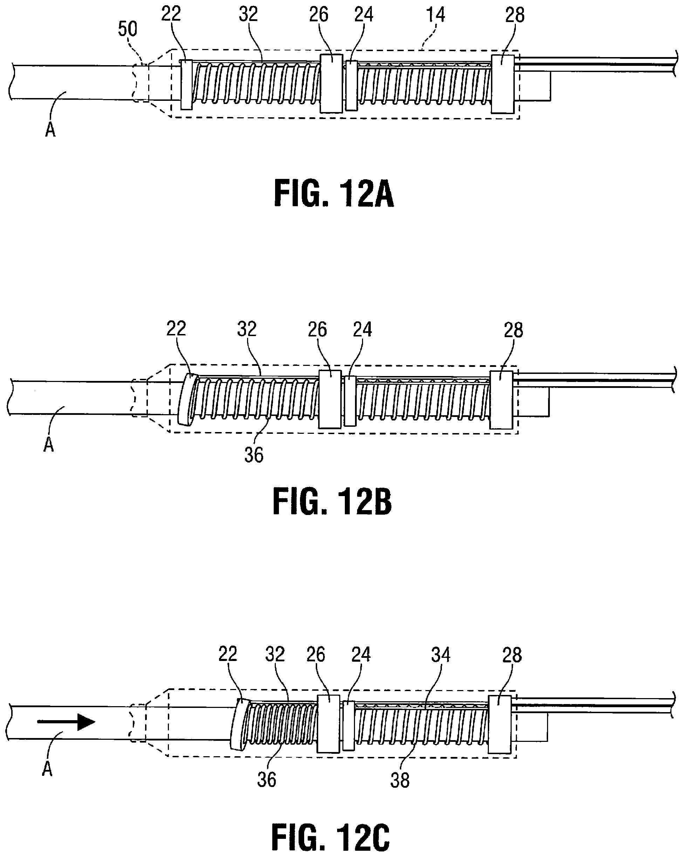

   Figure 12A illustrates the lead extractor in the neutral position with the proximal and distal clamp rings in the substantially perpendicular position;
   Figure 12B illustrates a first cable pulled proximally to move the distal clamp ring to the angled position;
   Figure 12C illustrates the first cable pulled further proximally to move the lead proximally;

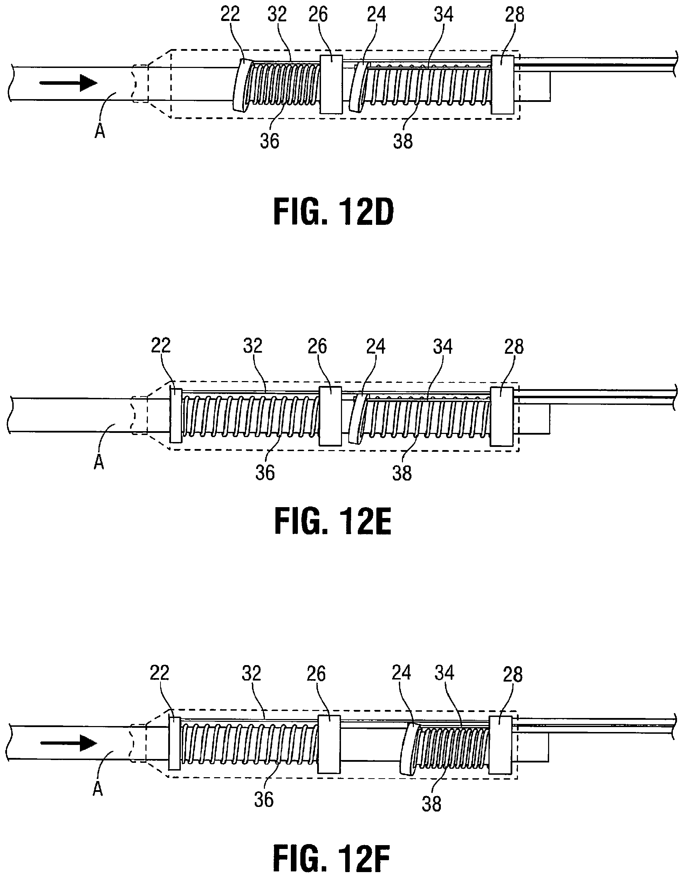

   Figure 12D illustrates a second cable pulled proximally to move the proximal clamp ring to the angled position;
   Figure 12E illustrates the first cable released to return the distal clamp ring to its substantially perpendicular and distal position;
   Figure 12F illustrates the second cable pulled further proximally to move the lead further proximally;

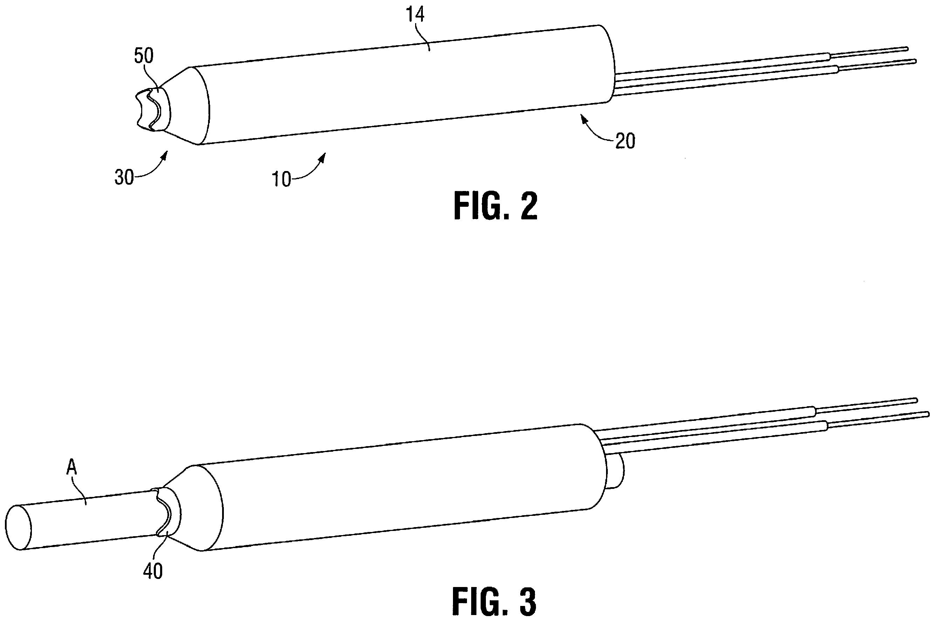

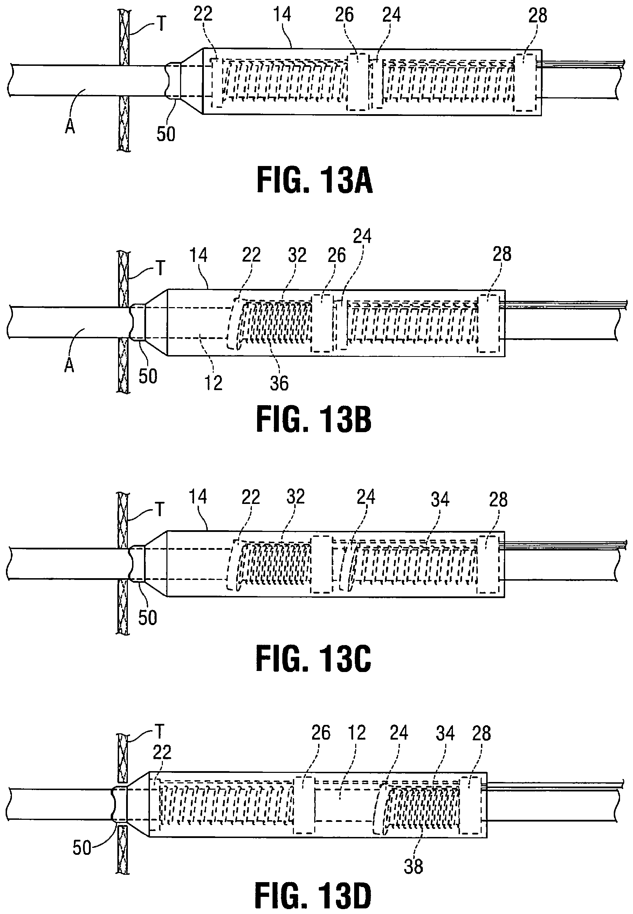

Figures 13A-13D are side views of the lead extractor of Figure 2, with the internal rings and spring shown in phantom, illustrating how the lead and extractor are relatively moved to cut tissue about the lead wherein:
   Figure 13A illustrates the lead extractor being inserted over an implanted lead to approach the tissue surrounding the lead, the lead extractor shown in the neutral position with the proximal and distal clamp rings in the substantially perpendicular position;
   Figure 13B illustrates the first cable pulled proximally to move the lead proximally and sever the surrounding tissue;
   Figure 13C illustrates a second cable pulled proximally to move the proximal clamp ring to the angled position;
   Figure 13D illustrates the first cable released to return the distal clamp ring to its substantially perpendicular position and the second cable pulled further proximally to move the lead further proximally with the surrounding tissue being severed;

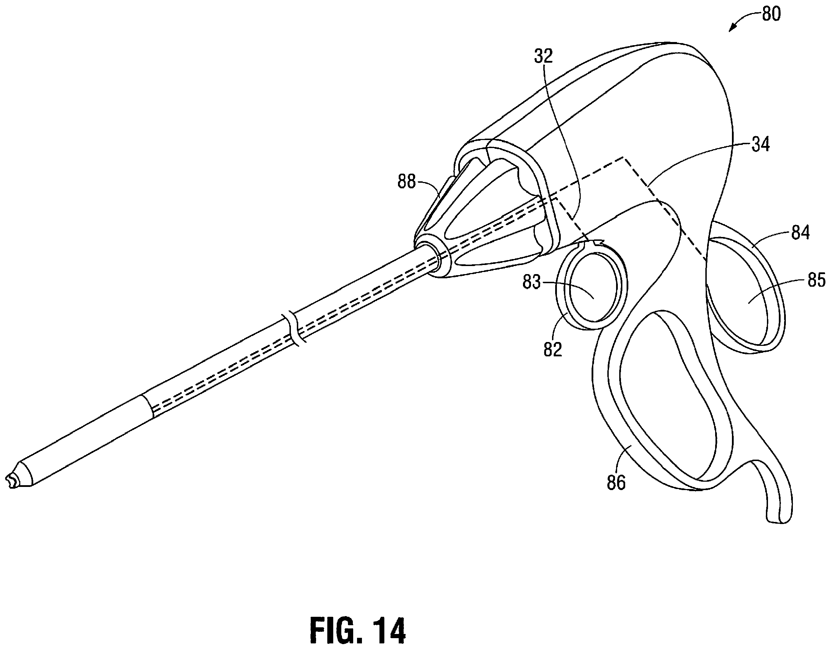

Figure 14 is a perspective view of an alternative embodiment not forming part of the present invention utilizing a manual control to actuate the cables of the extractor of Figure 2;

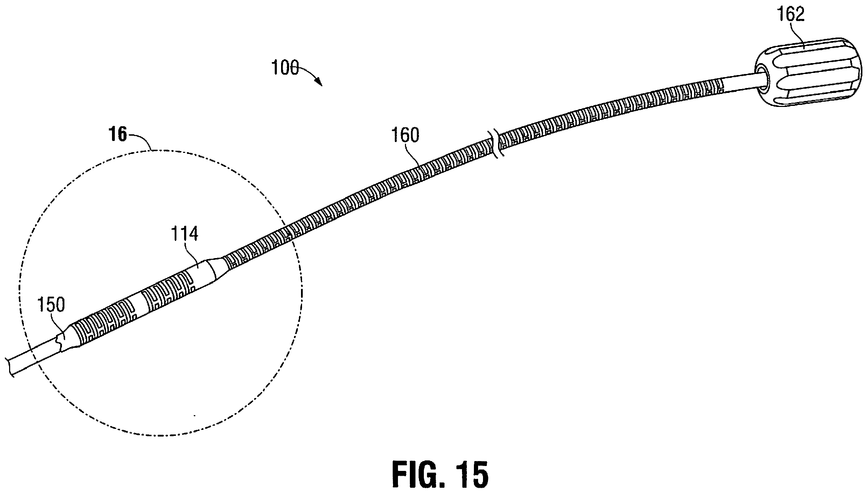

Figure 15 is a perspective view of an alternate embodiment of a lead extractor not forming part of the present invention having a flexible tube;

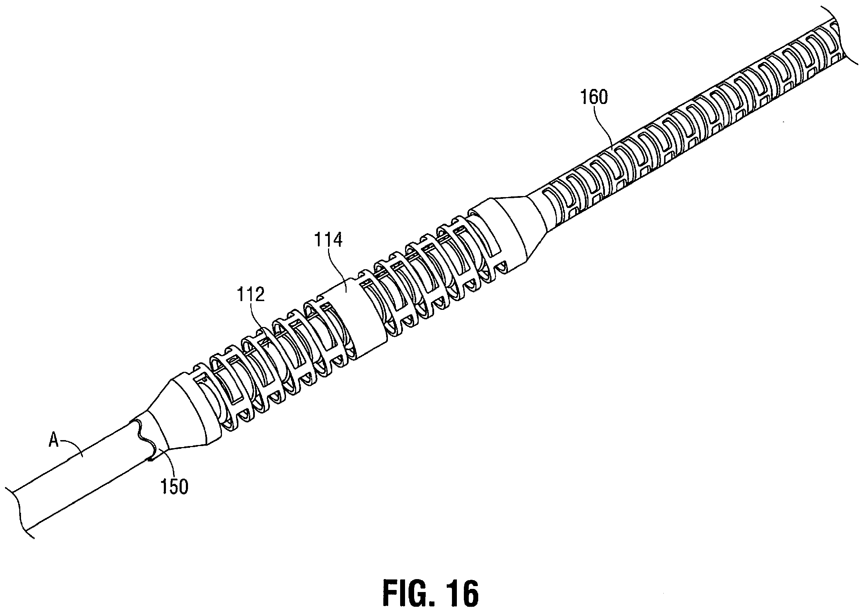

Figure 16 is a close up perspective view of the area of detail of Figure 15;

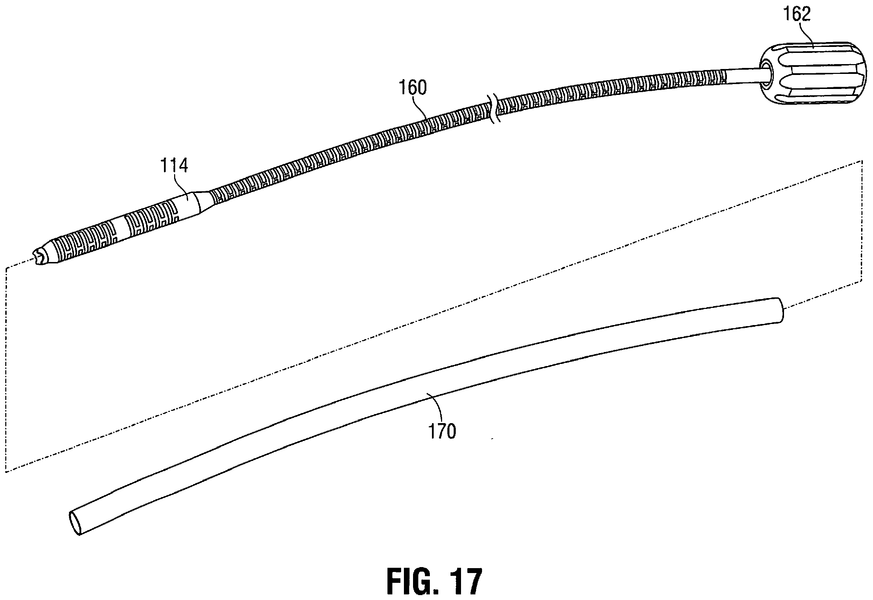

Figure 17 illustrates a perspective view of another alternate embodiment of a lead extractor not forming part of the present invention,

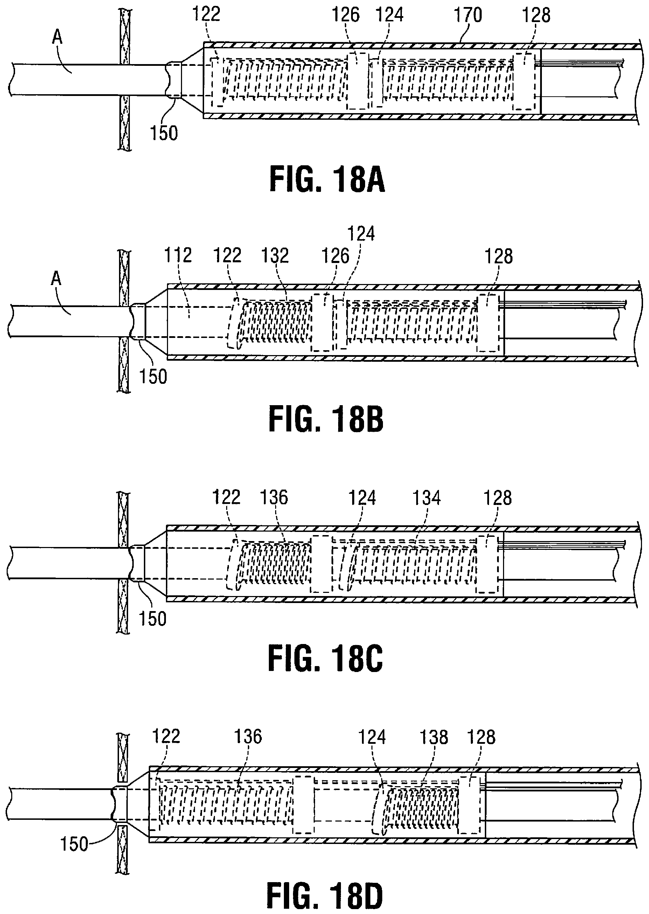

Figures 18A-18D are side views of the lead extractor of Figure 17, with the sheath shown in cross-section, illustrating how the lead and extractor are relatively moved to cut tissue about the lead wherein:
   Figure 18A illustrates the lead extractor being inserted over an implanted lead to approach the tissue surrounding the lead, the lead extractor shown in the neutral position with the proximal and distal clamp rings in the substantially perpendicular position;
   Figure 18B illustrates the first cable pulled proximally to move the lead proximally and sever the surrounding tissue;
   Figure 18C illustrates a second cable pulled proximally to move the proximal clamp ring to the angled position; and
   Figure 18D illustrates the first cable released to return the distal clamp ring to its substantially perpendicular position and the second cable pulled further proximally to move the lead further proximally with the surrounding tissue being severed.

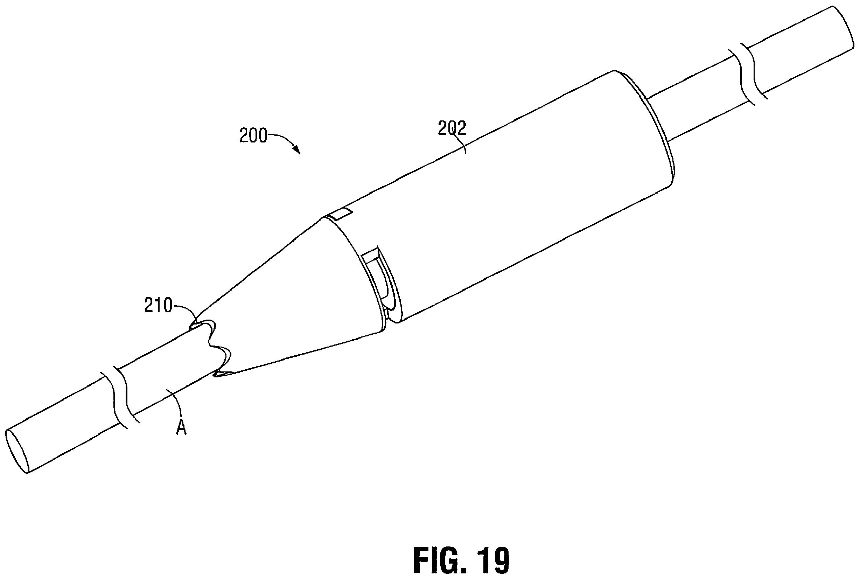

Figure 19 is a perspective view of an alternate embodiment of a lead extractor not forming part of the present invention shown positioned over a cardiac lead;

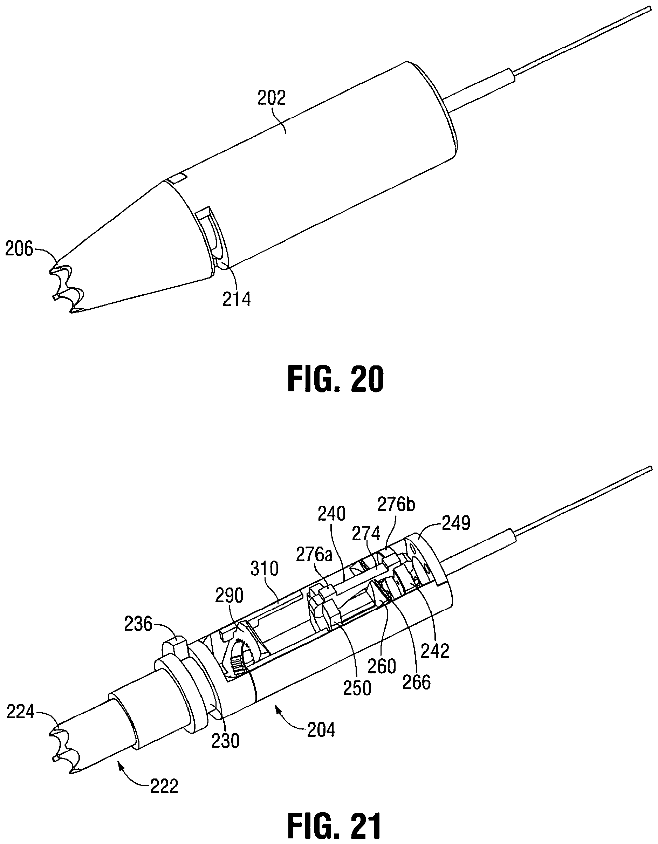

Figure 20 is a perspective view of the outer housing of the lead extractor of Figure 19;
Figure 21 is a perspective view of the inner housing of the lead extractor of Figure 19;

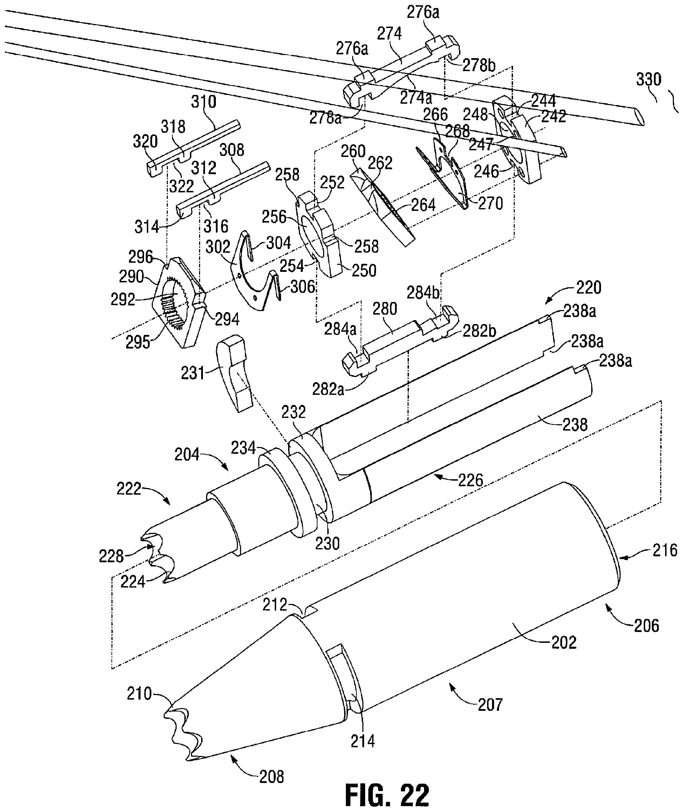

Figure 22 is an exploded perspective view of the lead extractor of Figure 19;

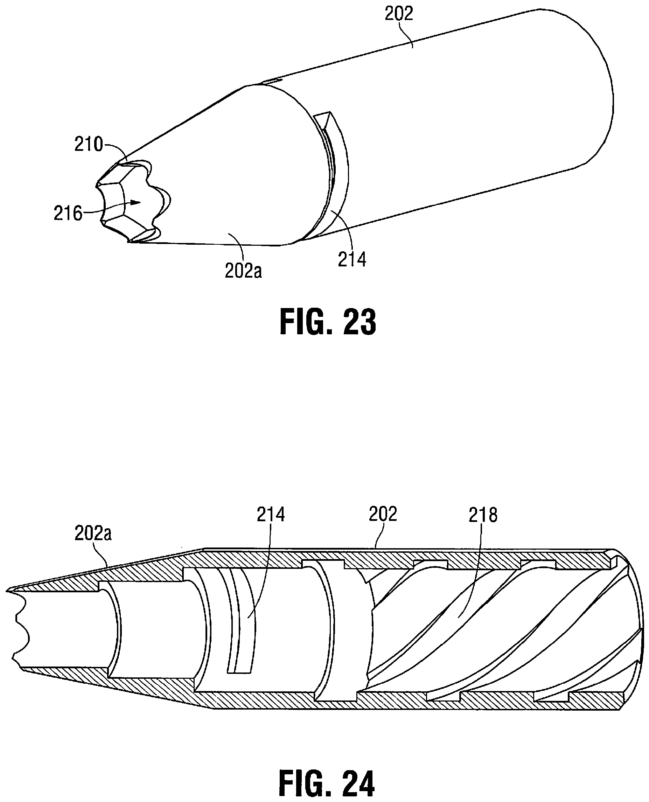

Figure 23 is a perspective view of the outer housing of the lead extractor of Figure 19;
Figure 24 is a cross-sectional view of the outer housing of Figure 23;

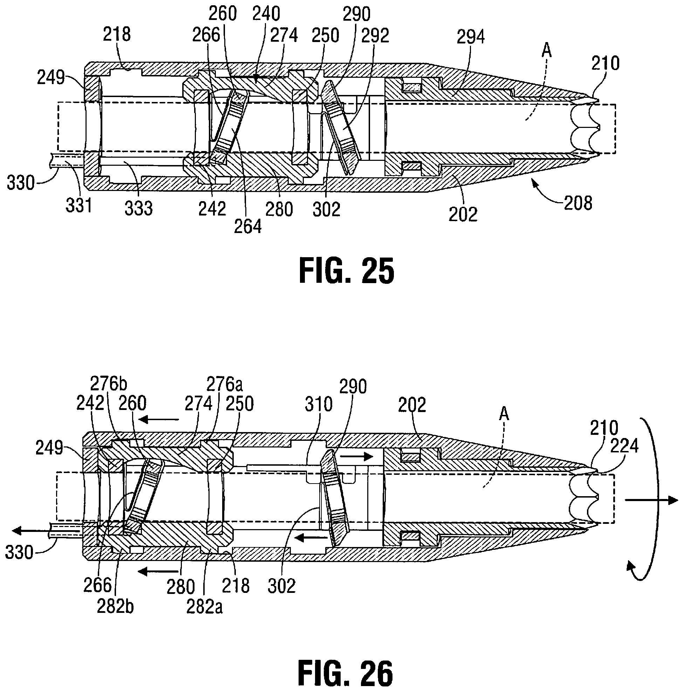

Figure 25 is a cross-sectional view illustrating the lead extractor of Figure 19 in the initial position and showing the lead extending through the extractor;
Figure 26 is a cross-sectional view similar to Figure 25 illustrating the carrier of the lead extractor moved to the proximal position;

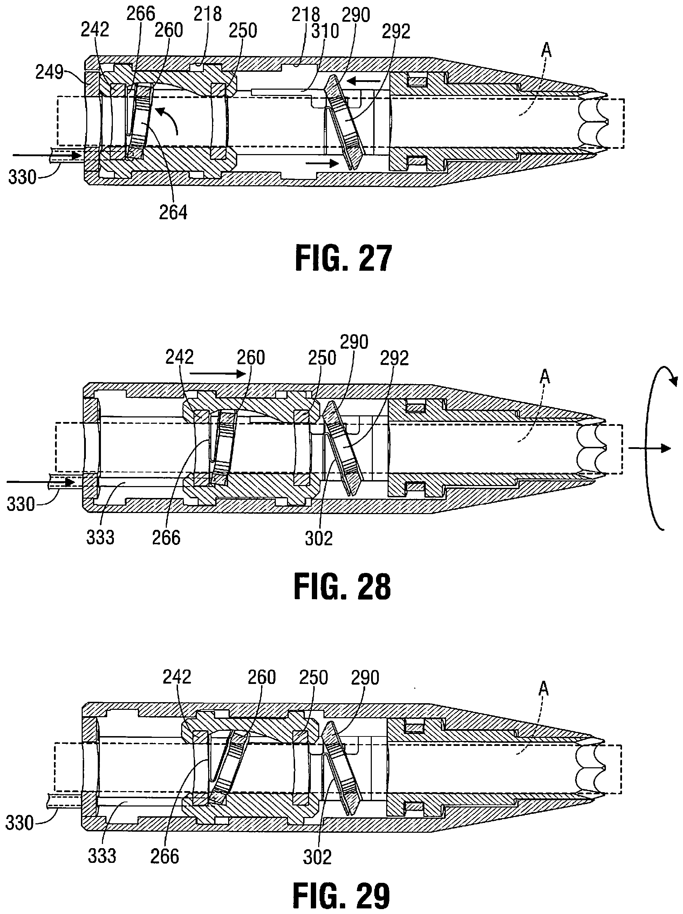

Figure 27 is a cross-sectional view similar to Figure 26 illustrating the carrier of the lead extractor starting to be returned to the initial distal position;
Figure 28 is a cross-sectional view similar to Figure 27 illustrating the carrier of the lead extractor moved to the distal position;
Figure 29 is a cross-sectional view similar to Figure 28 illustrating the carrier and components of the lead extractor in the initial position;

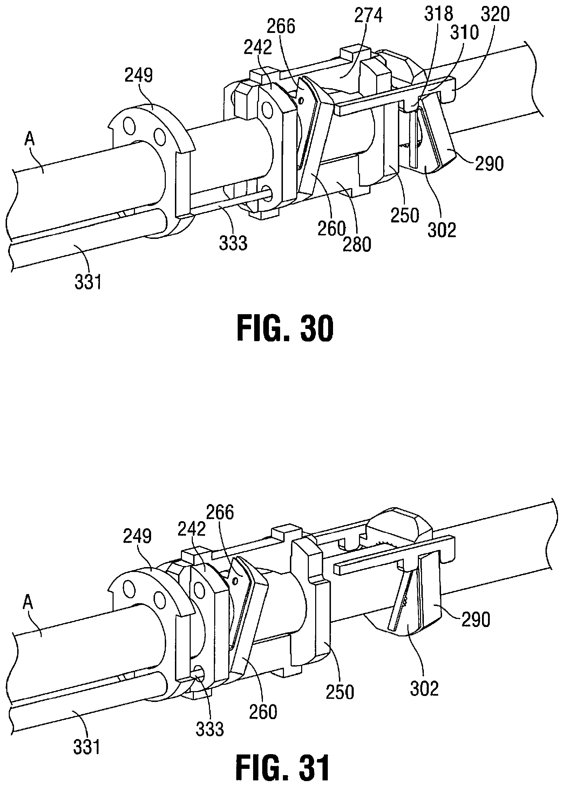

Figure 30 is a perspective view corresponding to the position of Figure 25, the inner and outer housings removed for clarity, and showing the lead extending through the extractor;
Figure 31 is a perspective view corresponding to the position of Figure 26, the inner and outer housings removed for clarity;

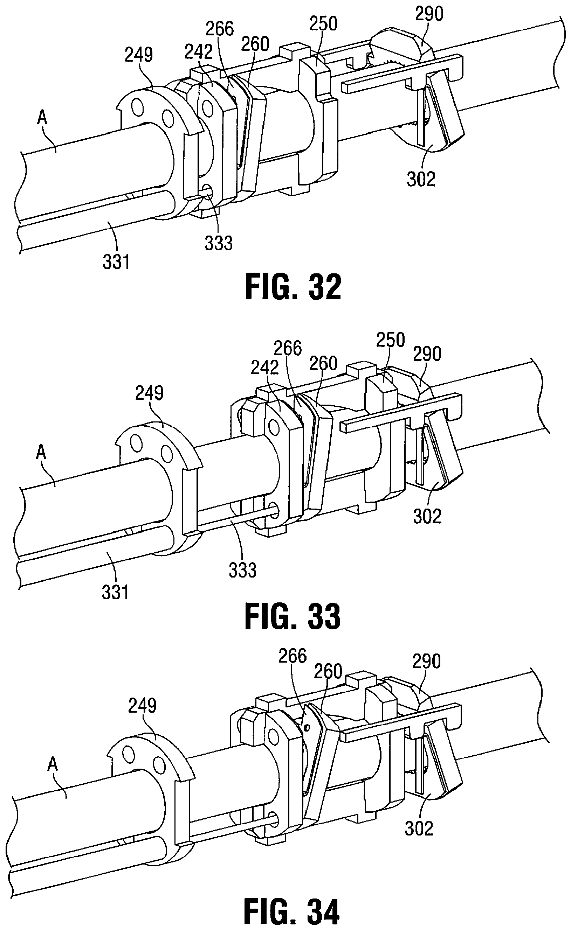

Figure 32 is a perspective view corresponding to the position of Figure 27, the inner and outer housings removed for clarity;
Figure 33 is a perspective view corresponding to the position of Figure 28, the inner and outer housings removed for clarity;
Figure 34 is a perspective view corresponding to the position of Figure 29, the inner and outer housings removed for clarity;

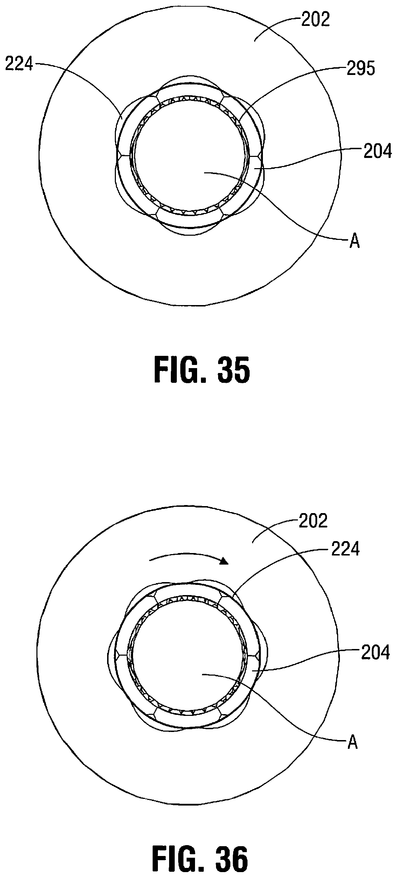

Figure 35 is a front view of the lead extractor of Figure 19;
Figure 36 is a front view similar to Figure 35 showing rotation of the outer housing with respect to the inner housing for cutting tissue;

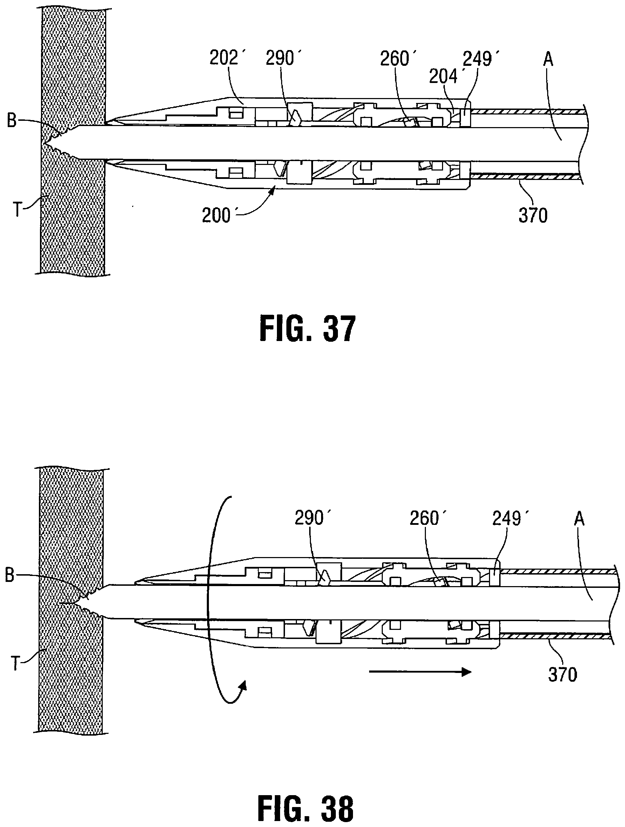

Figure 37 is a side view of an alternate embodiment of a lead extractor not forming part of the present invention having a flexible sheath thereover, the sheath shown in cross-section;
Figure 38 is a side view similar to Figure 37 showing rotation of the flexible sheath to rotate the extractor and lead;

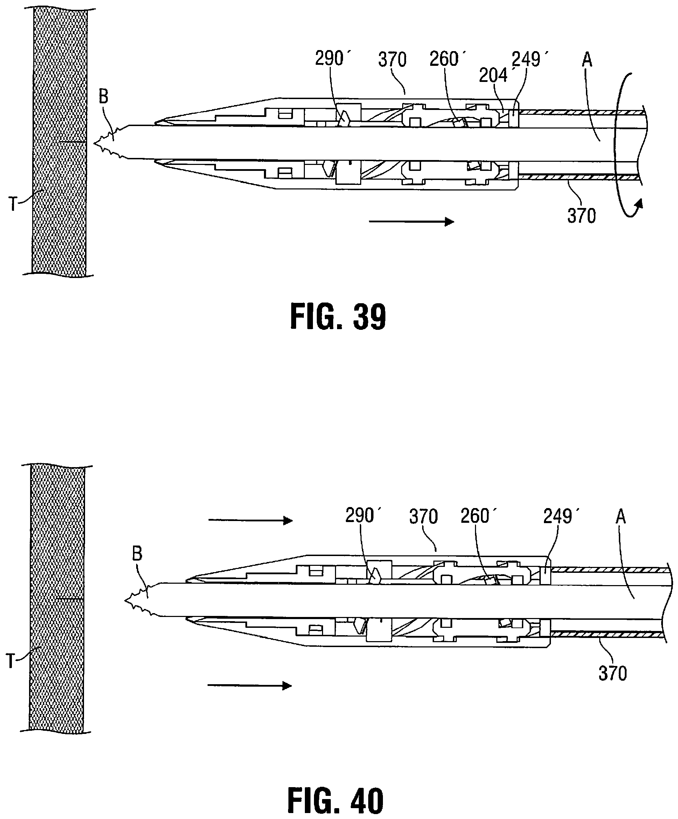

Figure 39 is a side view similar to Figure 38 showing freeing of the distal tip of the lead from the tissue as a result of rotation of the sheath;
Figure 40 is a side view similar to Figure 39 showing removal of the flexible sheath, lead extractor and lead from the body;

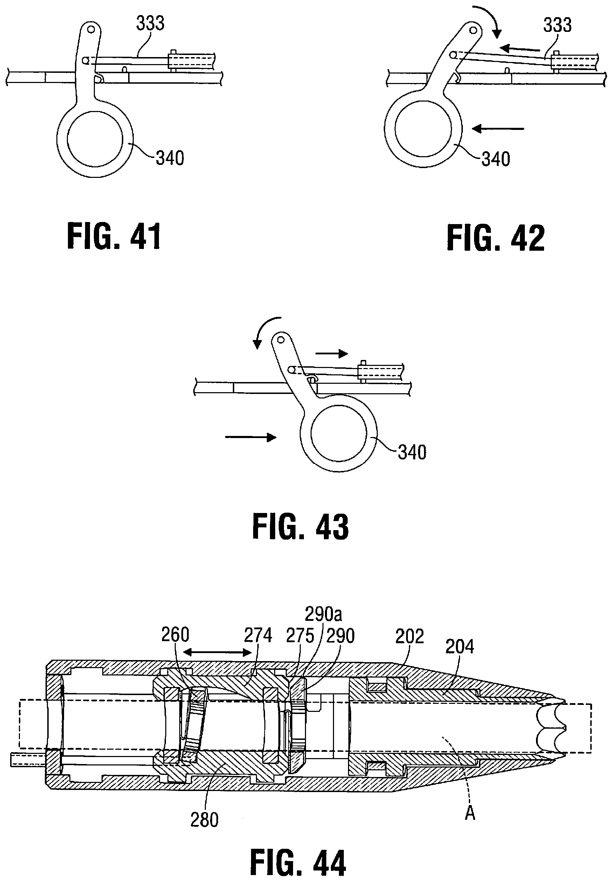

Figure 41 is a perspective view of an actuator not forming part of the present invention in a first (neutral) position;
Figure 42 is a perspective view of the actuator of Figure 41 in a second position to pull the cable proximally to retract the carrier of the lead extractor;
Figure 43 is a perspective view of the actuator of Figure 41 in an override position to advance the carrier of the electrode lead to the distal clamp release position;
Figure 44 is a cross-sectional view corresponding to Figure 19 illustrating the lead extractor in the override position to release the clamping rings;

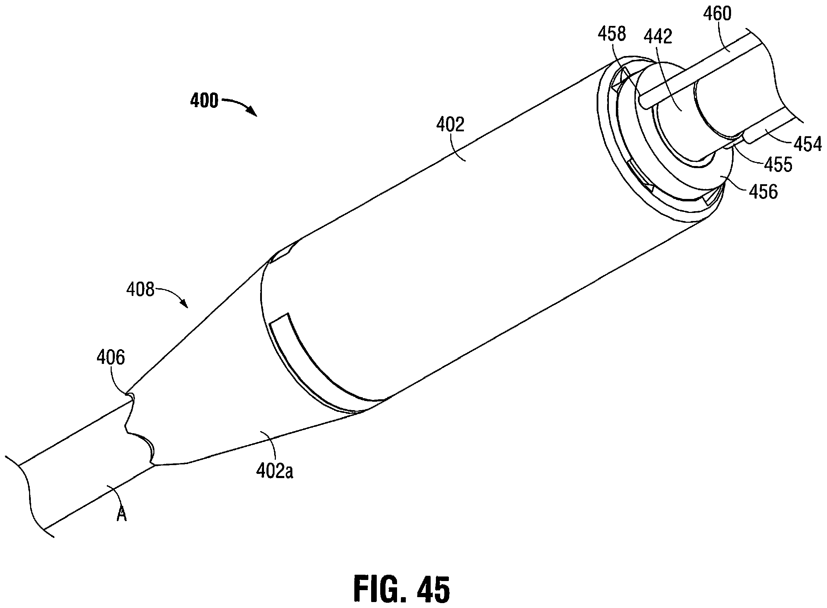

Figure 45 is a perspective view of an embodiment of a lead extractor of the present invention shown positioned over a cardiac lead;

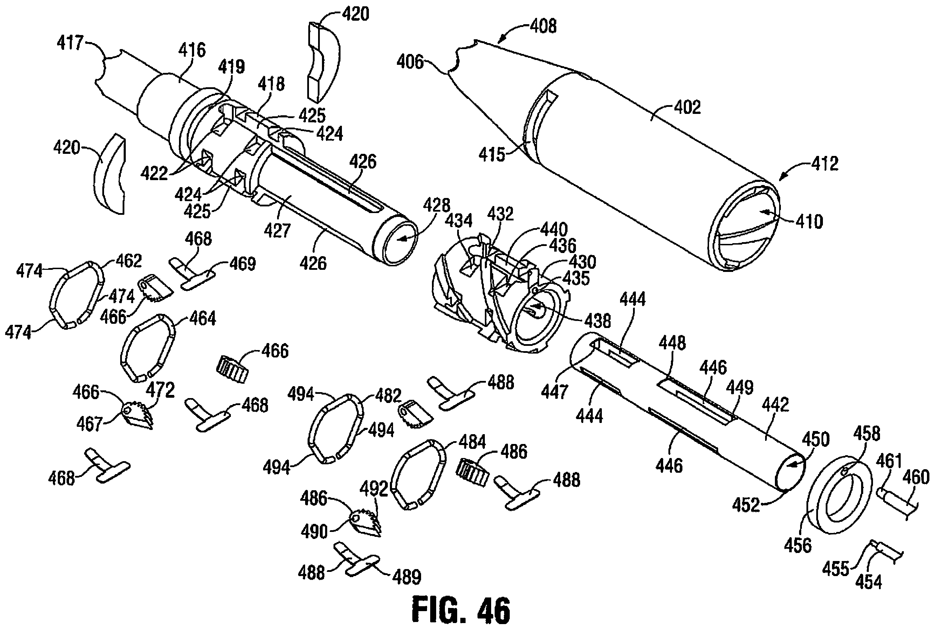

Figure 46 is an exploded view of the lead extractor of Figure 45;

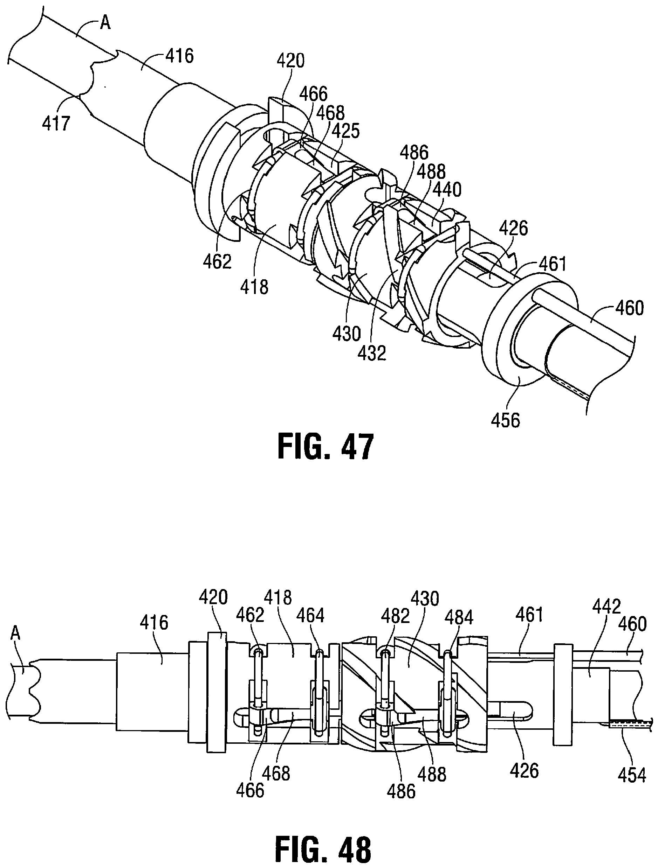

Figure 47 is a perspective view of the lead extractor of Figure 45 with the housing removed for clarity and the proximal jaw support (movable carrier) shown in the initial distal position;
Figure 48 is a side view of the lead extractor of Figure 47;

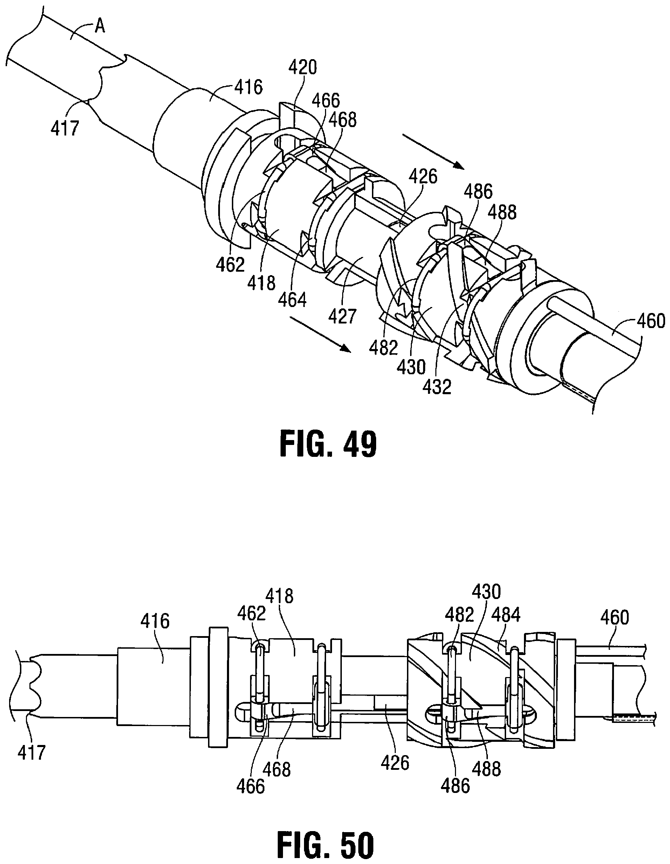

Figure 49 is a perspective view similar to Figure 47 showing the proximal jaw support in the proximal position;
Figure 50 is a side view of the lead extractor of Figure 49;

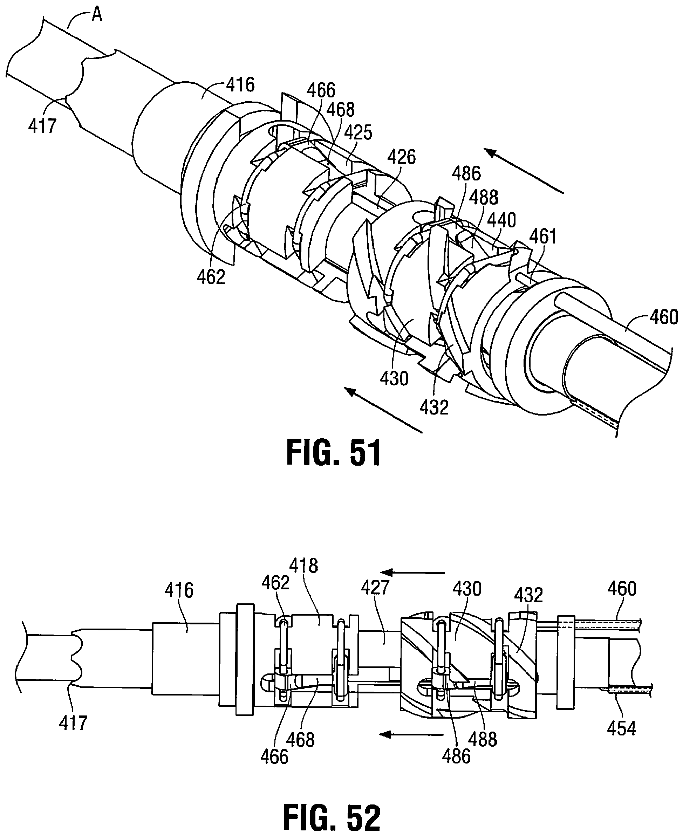

Figure 51 is a perspective view similar to Figure 49 showing the proximal jaw support being moved distally toward its distal position;
Figure 52 is a side view of the lead extractor of Figure 51;

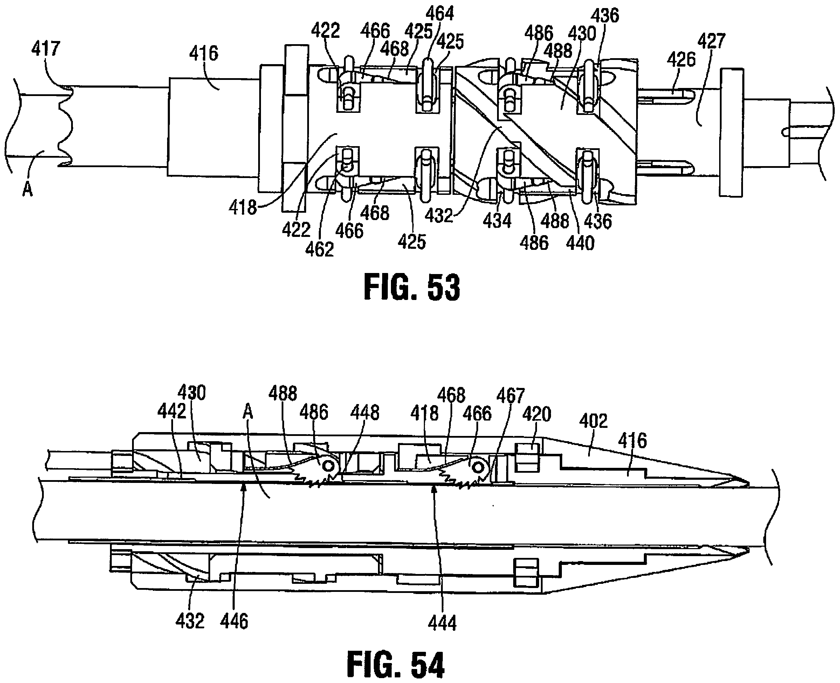

Figure 53 is a side view of the lead extractor in the position of Figure 48 showing the lead extractor from a different side;
Figure 54 is a sectional view showing the lead extractor in the position of Figure 48;

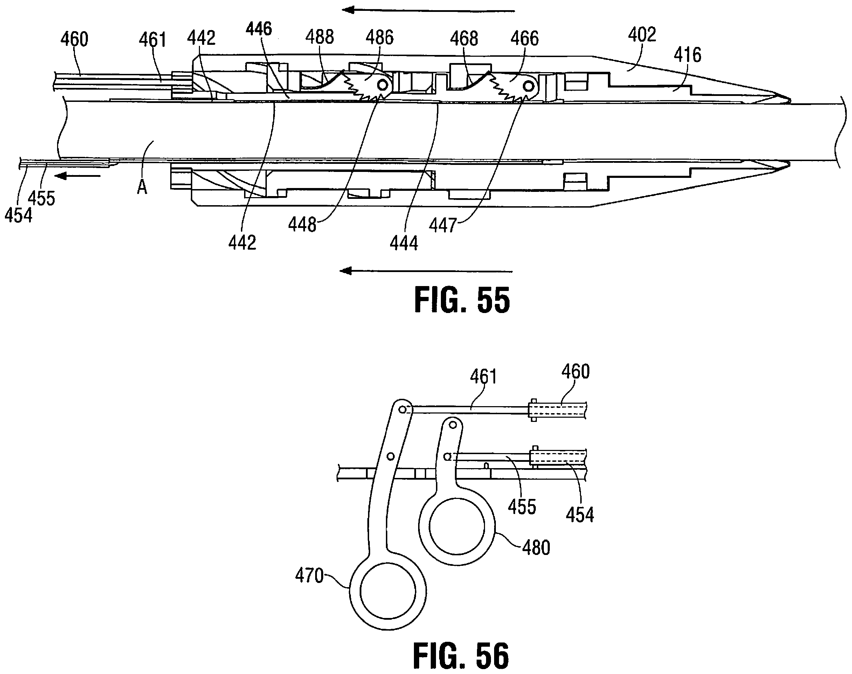

Figure 55 is a sectional view of the lead extractor of Figure 45 in the disengaged position to free it from engagement with the lead for removal; and
Figure 56 is a side view of an actuator for effecting movement of the proximal jaw support and the disengagement member of the lead extractor of Figure 45.

### DETAILED DESCRIPTION OF EMBODIMENTS

The lead extractors described herein advantageously hold the lead adjacent the area where the tissue cutting (severing and/or dissecting) occurs, thereby transferring the power of the work required to the location where it is needed. This provides an advantage over other lead extractors where the extractor is held and maneuvered from a proximal end to apply a cutting or dissecting force to the tissue at the remote distal end. Thus, the lead extractors of the present disclosure provides for lead removal with minimal damage to the patient's tissue.

To this end, the present disclosure describes lead extracting devices which grip and frictionally retain the lead, and then incrementally move relative to the lead, cutting the surrounding tissue as it is moved proximally within the device. Note the lead and extractor move relative to each other. That is, if the distal end of the lead is fixed, the relative movement will occur by the extractor being advanced along the lead. If the distal end of the lead is not fixed, relative movement will occur by the lead moving proximally within the extractor. Also, relative movement can include proximal movement of the lead simultaneous with distal movement of the extractor. In any case, as a result of this relative movement, the extractor "swallows" the lead within its lumen as it incrementally and progressively cuts through tissue around the lead to free the lead from the tissue. Cutting of tissue can occur by severing and/or dissecting tissue. The cutter is shown as part of the housing in the embodiments herein, however, alternatively the cutter can be a separate component attached to the housing.

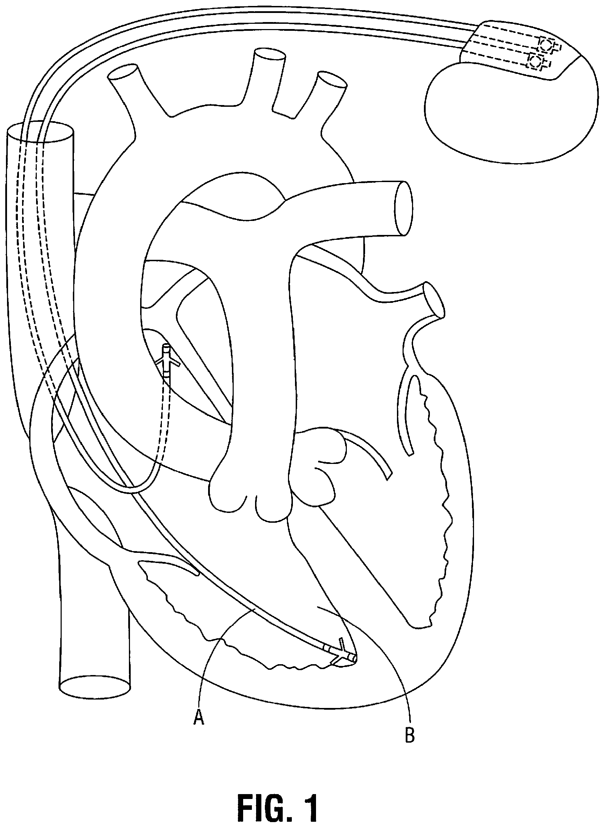

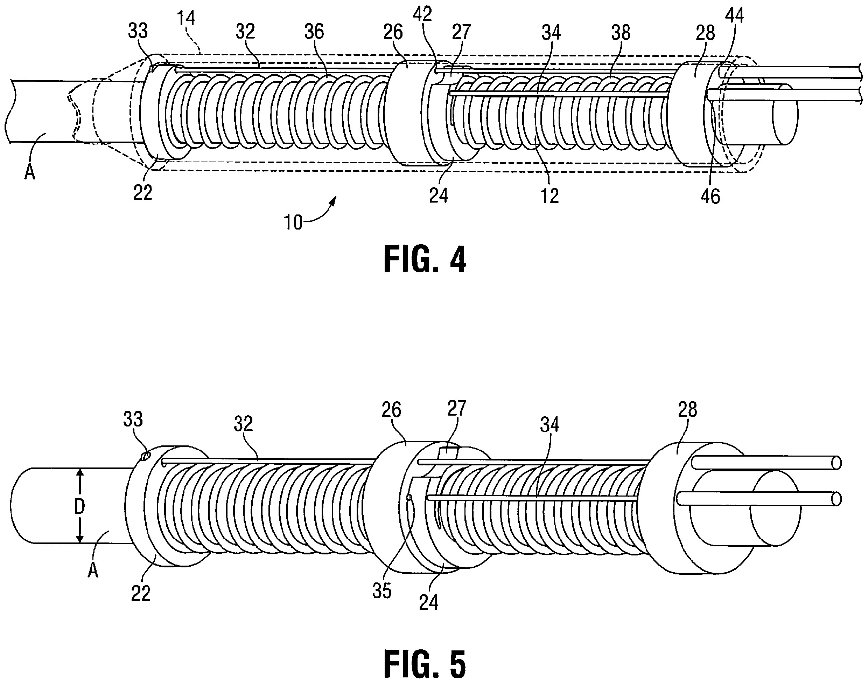

With reference to Figures 2, 4 and 5, a lead extracting device not forming part of the present invention is designated generally by reference numeral 10 and includes an outer housing or body 14 and an internal tubular member or inner body 12 having a lumen dimensioned to receive a lead therein. The outer housing (outer tube) 14 has a proximal end 20 and a distal end 30. As used herein the term "proximal" refers to the portion that is closer to the user and the term "distal" refers to the portion that is further from the user. A cutting knife (cutter) or cutting portion 50 is positioned at the distal end 30 of outer housing 14 configured to cut tissue surrounding the lead, designated by reference letter "A". Figure 1 illustrates an anatomical view of the heart, illustrating the location of the cardiac lead A which is desired to be removed from the right ventricle B. It should be appreciated that the devices disclosed herein are described for removing a cardiac lead, however, it should be understood that the devices also have other surgical applications. Note tissue ingrowth around the lead also occurs at regions along the length of the lead proximal of the distal tip of the lead.

The lead extractor includes a distal clamping ring 22, a proximal clamping ring 24, a distal fixed ring 26 and a proximal fixed ring 28. A first actuator or actuating (movement) mechanism in the form of a first wire or cable 32 is operably connected to the distal clamping ring 22 and a second actuator or actuating mechanism in the form of a second wire or cable 34 is operably connected to the proximal clamp ring 24. The cable 32 is operable to pivot distal clamping ring 22 from a substantially perpendicular position to an angled position with respect to the longitudinal axis of the extractor 10. In the substantially perpendicular position, the extracting device 10 is freely movable over the lead A. In the angled (tilted) or oblique position, due to the dimension of the opening in the distal clamping ring 22, the distal clamping ring 22 frictionally engages, i.e., clamps, the external surface of the lead A as the surface around the opening in the clamping ring 22 frictionally engages the outer surface of the lead. Such clamping allows relative movement of the lead, i.e., "swallowing" of the lead described in detail below. Similarly, the cable 34 is operable to pivot proximal clamp ring 24 from a substantially perpendicular position to an angled position with respect to the extractor 10. In the substantially perpendicular position, the extracting device 10 is freely movable over the lead A. In the angled (tilted) or oblique position, due to the dimension of the opening in the proximal clamping ring 24, the proximal clamping ring 24 frictionally engages, i.e., clamps, the external surface of the lead A as the surface around the opening in the clamping ring 24 frictionally engages the outer surface of the lead. Such clamping allows relative movement of the lead, i.e., "swallowing" of the lead as described in detail below. A distal spring 36 is positioned around tubular member 12 to bias the distal clamp ring 22 in a distal direction and a proximal spring 38 is positioned around tubular member 12 to bias the proximal clamp ring 24 in the distal direction.

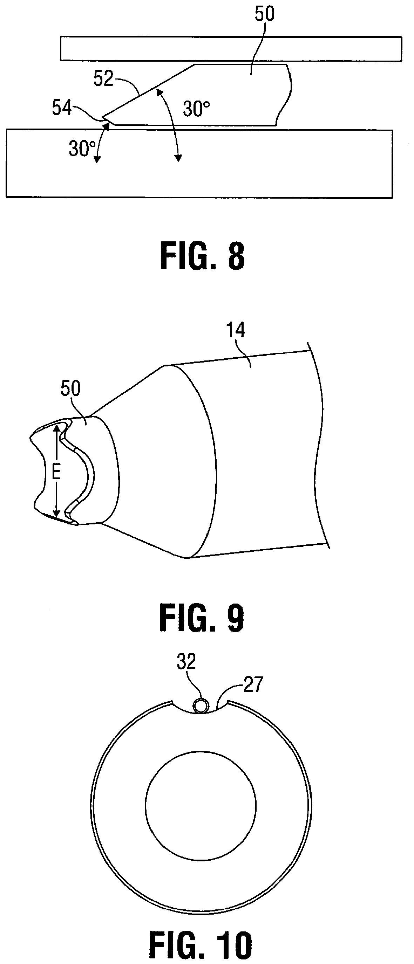

First cable 32, also referred to herein as the distal ring cable, is fixedly attached to distal ring 22 (at connection 33), extends through an aperture 42 in the distal fixed ring 26 and an aperture 44 in proximal fixed ring 28. Proximal clamp ring 24 has a cutout or notch 27 to accommodate the first cable 32 (see also Figure 10). The cable 32 extends proximally to a position outside the patient for manipulation manually by a user or alternatively for connection to a motor as described below.

Cable 34, also referred to herein as the proximal ring cable, is fixedly attached to proximal ring 24 (at connection 35) and extends through an aperture 46 in the proximal fixed ring 26. The cable 34 extends proximally to a position outside the patient for manipulation manually by a user or alternatively for connection to a motor. The cables 32 and 34 thereby provide a movement mechanism for the clamping members.

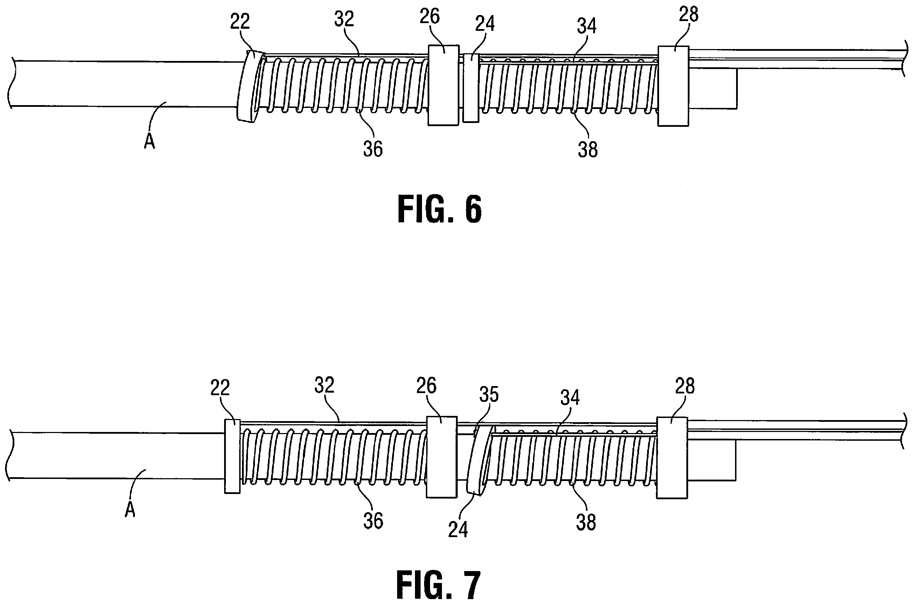

The extractor 10 preferably has three operable positions. In a first or initial position, referred to as the neutral or zero position, both the distal and proximal clamping rings 22, 24 are in the substantially perpendicular position in which they do not frictionally retain the cardiac lead and therefore the device 10 can be slidably moved over the lead A, as the lead A extends through the lumen of the tubular member 12. In this neutral position, this sliding movement is obtained since the inside diameter of the opening in the distal ring and the inside diameter of the opening in the proximal ring 22 is greater, e.g., slightly greater, than the outside diameter D of the lead A. Note this neutral position also enables the device 10 at any time during the procedure to release the lead and be adjusted or removed from the lead and patient. In the second position, the distal ring 22 is moved to the angled position to engage (clamp) the lead A while the proximal ring 24 remains in the substantially perpendicular position, as shown in Figure 6. In the third position, the proximal ring 24 is moved to the angled position to engage (clamp) the lead A while the distal ring 22 remains in the substantially perpendicular position, as shown in Figure 7. It should be appreciated that alternatively, the second position can denote when the proximal ring 22 is moved to the angled position to engage the lead A and the distal ring 24 remains in the substantially perpendicular position, in which case the third position would denote the position wherein the distal ring 22 is moved to the angled position to engage the lead A while the proximal ring 24 remains in the substantially perpendicular position.

The knife (cutter) 50 preferably has an angled cutting edge that avoids the knife cutting into the vessel wall. The cutting edge is beveled at end 52, and has a small cutting edge 54 (Figure 8), extending at an opposite angle to the bevel, thereby preventing the knife 50 from going into the lead. The knife 50 has a circular design with a sinuous shape, thereby providing a curved knife to perform a relative movement from the cutting edge to the tissue as in a guillotine-like action. The angles shown in Figure 8 are by way of example as other angles are also contemplated. The inner diameter E of the knife 50 (Figure 9) preferably is slightly greater than the outer diameter of the lead A to be received in the lumen of device 10.

Turning now to the method of use, and with reference to Figures 12A-12F, the device 10 is inserted over the lead A and advanced over the lead A until the knife 50 at the distal end 30 of the tubular member 12 is at the desired site, namely the site where the lead A is embedded or surrounded by tissue so it cannot be removed. This is typically proximal of the distal tip of the lead. In this position, the device 10 is ready for lead extraction.

The user then pulls cable 32 proximally, or if motor operated, turns on the motor which automatically pulls the cable 32 proximally. In the first proximal movement of the cable 32, the distal clamping ring 22 is pivoted to its angled position of Figure 12B to frictionally clamp or grasp the lead A. Upon further movement of the cable 32, the lead A is pulled back due to its frictional engagement with the device 10 via distal clamp ring 22 as shown in Figure 12C and/or the device 10 is moved over the lead in this relative movement to "swallow" the lead A. (Since the proximal ring 24 is in its substantially perpendicular or non-engaging position, the lead can move through the opening in the ring 24). As the lead A is moved back proximally in the direction of the arrow of Figure 12C it begins to be freed from tissue as the surrounding tissue is engaged and cut (severed and/or dissected) by knife 50. As can be appreciated, the cutting of tissue occurs adjacent the end of the device where the lead is engaged, thus providing more leverage and easier severing of the tissue. As the distal ring 22 is pulled proximally (rearwardly), it compresses distal spring 36. In an exemplary embodiment, distal clamping ring 22 is pulled back a maximum of approximately halfway to the distal fixed ring 26. In an exemplary embodiment, the distance between distal clamping ring 22 and distal fixed ring 26 is about 20 mm and the distal clamping ring 22 is pulled proximally about 10mm thereby moving the lead A proximally about 10 mm in the direction of the arrow of Figure 12C. Other distances are also contemplated.

Once the distal clamping ring 22 has been pulled back to relatively move the lead A proximally or "swallow" the lead a predetermined amount, the second cable 34 can now be actuated. The user pulls cable 34 proximally, or if motor operated, the motor automatically pulls the cable 34 proximally after the first cable 32 has been pulled. In the first proximal movement of the cable 34, the proximal clamping ring 24 is pivoted to its angled position of Figure 12D to frictionally clamp or grasp the lead A. Once the cable 34 has moved the proximal ring 32 to its angled position, the tension on the first cable 34 is released so the distal ring 22 can return to its substantially perpendicular or non-engaged position, aided by the force of distal spring 36 (Figure 12E). In a preferred embodiment, the first cable 32 (and thus the distal clamp ring 22) is not released until the second cable 34 has been tensioned to move the proximal ring 24 to engage the lead A. This helps prevent slippage, e.g., distal movement of the lead A, since the lead A is continuously being grasped, albeit by alternating the grasping function between the proximal and distal clamping rings 22, 24.

After the cable 34 has been pulled to pivot the proximal clamping ring 24 to its angled position, further retraction of the cable 34 pulls the lead A back (proximally) or moves the device 10 distally due to its frictional engagement with the device 10 via distal clamp ring 24. Thus, the lead A is relatively moved further back proximally in the direction of the arrow to further free it from surrounding tissue as the tissue is cut by knife 50 as shown in Figure 12F. As the proximal ring 22 is pulled proximally (rearwardly), it compresses proximal spring 38. In an exemplary embodiment, proximal ring 22 is pulled back a maximum of approximately halfway to the proximal fixed ring 28. In an exemplary embodiment, the distance between proximal clamp ring 24 and proximal fixed ring 28 is about 20 mm and the proximal clamp ring 24 is pulled proximally about 10mm, thereby relatively moving the lead A about 10 mm. Other distances are also contemplated.

Next, the first cable 32 is pulled to once again pivot the distal ring 22 to the angled engaging position. Once pulled, the second cable 34 can now be released, followed by further retraction of the first cable 32, to move the lead proximally due to its frictional engagement. After such movement, the second cable 34 is pulled proximally, followed by release of the first cable 34, and then further pulling of the cable 34 to move the lead A still further proximally and to continuously sever the surrounding tissue by knife 50. This step of alternatively pulling of the cables 32, 34 is repeated until the lead A is freed from the tissue and can be removed (with or separately from the device 10) from the tissue. This alternating cable motion can also be referred to as an oscillating movement in that the pulling of the cable alternates between the first and second cables, to incrementally pull the lead proximally or advance the extractor distally. This alternating action can also be considered as a step by step progressive "swallowing" of the lead. It can also be considered a tunneling action as it tunnels through tissue to separate tissue from the lead.

Figures 13A-13D illustrate how the lead is pulled back relative to tissue T. Figure 13A shows the lead extractor being inserted over lead A to the position where the lead is captured by tissue T. After positioning of the extractor 10 in the desired position, the first cable 32 is pulled rearwardly and the lead A is retracted or the device 10 advanced as described above, with the tissue T engaging the cutting edge of knife 50 to cut the tissue surrounding the lead A to thereby free the lead (see Figure 13B). In Figure 13C, the proximal clamp ring 24 is angled by the pulling of the second cable 34. The first cable 32 is then released, and the proximal cable 34 is pulled back further to further move the lead proximally or advance the device 10, thereby causing the tissue to again be into contact with the knife 50 to cut the tissue. As explained herein, this keeps being repeated so that the knife 50 can continue to cut the tissue as the lead A is incrementally and progressively pulled rearwardly or swallowed within the lumen of tube 12 of the lead extractor 10.

In one embodiment, this alternating movement can be achieved by handle mechanism 80 shown in Figure 14. The handle mechanism 80 includes a first actuator 82, illustratively in the form of a pivotable handle with a finger loop 83, and a second actuator 84, also illustratively in the form of a pivotable handle with a finger loop 85. The first actuator 82 is operatively connected to the first cable 32 such that proximal movement of the actuator 82, e.g., moving of the finger loop 83 toward stationary handle 86, pulls the cable 32 proximally and distal movement returns the cable 32 to its original position. Similarly, second actuator 84 is operatively connected to the second cable 34 such that proximal movement of the actuator 84 (away from the stationary handle 86) pulls the cable 34 proximally and distal movement returns the cable 34 to its original position. The handle mechanism 80 in a preferred embodiment includes a locking mechanism (not shown) to ensure that either cable 32, 34 cannot be released until the other cable has been moved proximally to move its respective clamping ring to the angled clamping position to frictionally engage the lead. A rotation knob 88 can be provided to rotate the lead extractor to thereby rotate the clamped lead if desired.

As can be appreciated, the pistol grip and pivotable handles are shown by way of example as other handle configurations and other types of actuators, e.g., sliding tabs, are also contemplated to provide manual control of the cable movement.

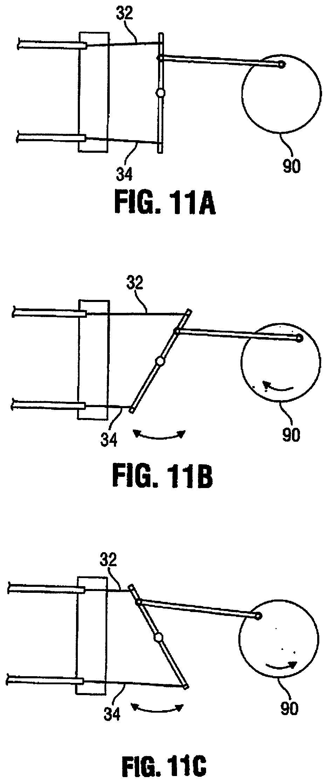

In an alternate embodiment, an external power source such as a motor assembly is provided to electrically drive (actuate) the cables instead of the manual operation by the user. As shown schematically in Figures 11A, motor rotation of the wheel 90, which is preferably eccentric, from the neutral position of Figure 11A to the position of Figure 11B, pulls cable 32 proximally to move the distal clamping ring 22 to the angled position and then to pull the distal clamping ring 22 proximally to retract or swallow the clamped lead as described above. Rotation of the wheel in the opposite direction (Figure 11C) will cause cable 34 to be pulled proximally to move the proximal clamping ring 24 to the angled position and to pull the proximal clamping ring 24 proximally to retract the clamped lead further proximally or further swallow lead. Thus, as can be appreciated, the motor causes the oscillating motion of the wheel and respective cables to incrementally relatively retract the lead. It should be appreciated that the motor controlled embodiment can be configured so that the clamping ring cannot be released from its angled position until the other clamping ring is moved to its angled clamping position as described above. Note that such motor operated cables can be utilized with the other embodiments disclosed herein, e.g., extractor 200 discussed below.

Figures 15-18 illustrate an alternate embodiment of a lead extractor not forming part of the present invention, designated generally by reference numeral 100. The lead extractor 100 is identical to the lead extractor 10 of Figure 2 except for provision of the flexible tube/sheath. Therefore, the identical components have been labeled with corresponding numbers in the "100 series" so that extractor 100 has a knife (cutter) 150, distal clamping ring 122, a proximal clamping ring 124, a distal fixed ring 126, a proximal fixed ring 128, a first cable 132 operably connected to the distal clamp ring 122 and a second cable 134 operably connected to the proximal clamp ring 124. The cable 132 is operable to pivot distal clamping ring 122 from a substantially perpendicular position to an angled position and the cable 134 is operable to pivot proximal clamping ring 124 from a substantially perpendicular position to an angled position. As in the embodiment of Figure 2, a distal spring 136 is positioned around tubular member 112 to bias the distal clamping ring 122 in a distal direction and a proximal spring 138 is positioned around tubular member 112 to bias the proximal clamping ring 34 in the distal direction.

The extractor 100 differs from extractor 10 in that a flexible tube (sheath) 160 having a handle 162 is provided. The handle 162 enables the extractor 100 to be rotated to thereby rotate the clamped lead. Such rotation provides an unscrewing action of the lead if the user deems it desirable. Thus, after the extractor 100 cuts the tissue surrounding the lead, the user can keep the extractor 100 locked to hold the lead, and the sheath can be rotated to facilitate removal of the embedded screwed-in tip of the lead. Note the tube 160 has a plurality of cutouts in the wall to provide the desired flexibility. The housing 114 can also have a plurality of cutouts in the wall to provide the desired flexibility. In the alternate embodiment of Figure 17, a sheath 170 is provided to cover the flexible tube 160.

Figures 18A-18D illustrate the use of extractor 100 which is identical to the use described in conjunction with Figures 13A-13D except for provision of a flexible sheath 170 in which the housing 114 is positioned. Thus, as can be appreciated the movement of the clamping rings 122 and 124, and relative movement of the extractor 10 and the lead A shown in Figures 18A-18D are identical to that of Figures 13A-13D and for brevity are not repeated herein.

Although two clamping rings are described in the embodiments herein, it is also contemplated that a single clamping ring or more than one clamping ring can be utilized.

An alternate embodiment of a lead extractor not forming part of the present invention is illustrated in Figures 19-36. The lead extractor is designated generally by reference numeral 200 and includes an outer body (outer housing) 202 and an inner body (inner housing) 204. The lead extractor 200 is similar to lead extractor 10 described above in that it is configured to move relative to the lead to "swallow" the lead in increments. However, in the lead extractor 10 of Figure 2, the user manipulates the actuators to selectively control pivoting of the clamping members, alternately clamping and releasing the distal and proximal clamping members. In the lead extractor 200 of Figure 19, the changed orientation of the clamping members is a result of the relative movement of the lead extractor 200 and lead. Additionally, the lead extractor 200 has an enhanced cutting action as the cutter also rotates. Other differences between extractor 200 and extractor 10 will become apparent from the detailed description below of extractor 200.

Turning to the components of lead extractor 200, and with reference to Figures 20-24, outer body (or outer tube) 202 of lead extractor 200 has a proximal portion 206, a distal portion 208 and an intermediate portion 207 therebetween. A cutter or cutting portion 210 is formed at the distal portion 208 and preferably includes a serrated edge or toothed edge to effectively cut tissue adjacent the lead. Outer body 202 is positioned coaxially over inner housing (or inner tube) 204 as the inner housing 204 is received in lumen 216 of outer body 202. Outer body 202 has an internal helical slot 218 and is rotatable relative to inner housing 204 (see Figures 35 and 36) to cut (sever and/or dissect) tissue as described in more detail below. Outer housing 202 has a conical tip 202a tapering in a distal direction to facilitate tunneling of the device. Radial slots 212, 214 receive disc 231 and another disc (not shown) or bars which are welded to the outer tube 202 to keep the device 200 together. Also, these block axial movement of the outer body 202 so that when the carrier 240 moves axially, since axial movement of the outer body 202 is blocked, it is forced to rotate.

With reference to Figures 21-22, the inner housing 204 has a proximal portion 220, a distal portion 222 and an intermediate portion 226 between the proximal portion 220 and distal portion 222. A cutter or cutting portion 224, preferably having a serrated or toothed edge as shown, interacts with the cutting portion 210 of outer housing 202 to sever tissue adjacent the lead. That is, the cutting portion 210 of outer housing 202 overlies a counterpart cutting portion 224 of inner housing 204. A circumferential slot 230 is formed between ring 234 and distal end 232 of the carrier receiving portion to receive semicircular disc 231.

Inner housing 204 has a pair of proximally extending arms 238 to form a gap to slidably receive carrier or vehicle 240. Movement of carrier 240 effects relative movement of the extractor 200 and lead. A proximal end cap 249 is secured within top and bottom notches 238a of arms 238 to secure the arms 238 and provide a back wall enclosure for the inner housing 204. Carrier 240 is slidably mounted within inner housing 204 for movement between proximal (retracted) and distal positions, proximal defined as noted above as the region closer to the user and distal as the region further from the user (and closer to the tip of the lead). The movement of carrier 240 provides the desired clamping of the lead which is positioned within the lumen 228 of inner housing 204. A cable 330 described in detail below effects movement of the carrier 240.

Carrier 240 is formed by proximal fixed support ring 242, distal fixed support ring 250, upper support 274 and lower support 280. The terms "upper" and "lower" as used herein refer to the orientation of the device in the orientation shown in the drawings and are used herein for ease of description. Clearly, if the orientation of the device changes, the references "upper" and "lower" will also accordingly change. Contained within carrier 240 is proximal clamping ring 260 which has a hinge point on its lower surface and is biased by proximal spring 266 to a tilted position (with respect to the longitudinal axis of the extractor 200 and lead) as shown in Figures 21 and 25. In this tilted position (tilted toward the distal end of the device), the proximal clamping ring 260 provides a clamping force on the lead as its central opening 264 is sufficiently angled with respect to the outer surface of the lead so the surface surrounding opening 264 grasps (clamps) the lead. Upper support 274 has a distal notch 278a seated within upper notch 252 of distal fixed support ring 250 and a proximal notch 278b seated within upper notch 244 of proximal fixed support ring 242 to retain and secure these components. Similarly, lower support 280 has a distal notch 284a seated within lower notch 254 of distal fixed support ring 250 and a proximal notch 284b seated within lower notch 246 of proximal fixed support ring 242 to retain and secure these components. Upper tabs 276a, 276b of upper support 274 and lower tabs 282a, 282b of lower support 280 interact with the helical slot 218 formed in the outer housing 202 described in more detail below. Proximal clamping ring 260 receives elongated portion 274a of upper support 274 in upper slot 262. A similar slot on the opposing (bottom) side of proximal clamping ring 260 receives lower support 280. A slot 268 of proximal spring 266 accommodates upper support 274. Spring 266 is hinged at a bottom portion and has an opening 270 through which the lead can extend. Spring 266 is preferably attached to proximal clamping ring 260.

Distal of carrier 240, positioned within inner housing 202 between arms 238, is a distal clamping ring 290 which has a hinge point on the top surface and is biased by distal spring 302 to the tilted position as shown in Figures 21 and 25. Spring 302 is preferably attached to distal clamping ring 290 and hinged at a top portion. As can be appreciated, the distal clamping ring 290 and proximal clamping ring 260 have hinge points on opposing sides of the longitudinal axis of the device 10. In the tilted position of Figure 21, (tilted toward the proximal end of the device), the distal clamping ring opening 290 is at a sufficient angle with respect to the lead such that the lead is clamped by the ring 290. A ridged, toothed or irregular surface 295 is formed around part or alternatively the entire circumference of opening 292 in distal clamping ring 290 to enhance clamping of the lead extending therethrough when the distal clamping ring 290 is in the tilted position. Such ridged, toothed or irregular surface can also be provided around part or the entire circumference of the opening 264 of proximal clamping ring 260 to enhance clamping of the lead.

Clamp engaging member 308 has a distal tab 314 and proximal tab 312. Clamp engaging member 310 similarly has a distal tab 320 and a proximal tab 318. The clamp engaging members 308, 310 are seated within side notches 294, 296, respectively, of distal clamping ring 290. The tabs 314, 312, 318, and 320 support and retain the upper end of the distal clamping ring 290.

Cable 330 (Figure 25) includes an outer cable 331 which is attached to the end cap 249 of inner housing 204. Coaxially positioned within the outer cable 331 is inner cable 333 which extends distally from outer cable 331 and is attached to the fixed proximal ring 242 of carrier 240. Cable 333 provides a movement mechanism as proximal movement of inner cable 333 pulls the carrier 240 in a proximal direction and distal movement of the inner cable 333 pushes the carrier 240 in a distal direction. The cable 333 is actuated by a trigger 340 shown in Figure 41 and described in conjunction with the method of use.

The use of the extractor 200 will now be described for use to extract an implanted cardiac lead, it being understood it can be used to extract other leads or other components/devices. Oftentimes, tissue ingrowth and plaque builds around the lead over a period of time which makes extraction difficult. The extractor 200 functions to extract the lead by application of the force at the distal end. That is, the lead extractor 200 is advanced in steps (increments) relative to the lead, thereby cutting e.g., severing and/or dissecting, tissue about the lead and tunneling around the lead to cut it away from tissue. When the tissue has been cut away, the lead can be extracted from the heart tissue. The extractor 200 and lead A are relatively movable with respect to each other. Therefore, if the lead is fixed, then the extractor 200 will move progressively (in discrete increments) over the lead; if the lead is not fixed, then the extractor will progressively pull the lead (in discrete increments) back into the extractor 200. Alternatively, both the extractor and lead can move in opposing directions. In any event, this relative movement causes the "swallowing" of the lead by the extractor 200.

Figures 25-29 show in cross-sectional views operation of the extractor 200. Figures 30-34 are perspective views corresponding to the respective positions of Figures 24-29, however the inner housing 204 and outer housing 202 have been removed for clarity.

In use, the device 200 is inserted over a proximal end of the lead, e.g., a cardiac lead, which is embedded in tissue and desired to be removed. The extractor 200 is advanced until the distal end 208 of the outer housing 202 encounters hard tissue. Note, in the insertion position, the proximal clamping ring 260 is tilted toward the distal end and the distal clamping ring 290 is tilted toward the proximal end as shown in Figures 25 and 30. In this position, the extractor 200 can be forced over the lead A with the openings 292 and 264 of distal and proximal clamping rings 290, 260 providing a sufficient gap (upon such force being applied) for passage of the outer diameter of the lead and not providing a sufficient clamping or frictional force on the lead A to prevent such passage. In this initial position for insertion over the lead, springs 266 and 302 are not compressed and bias the clamping rings 260, 290, respectively in the tilted positions shown. Note the clamping rings 260, 290 can optionally be moved to a less tilted position by movement to the override position described below for initial insertion of the lead, however, in this embodiment it is not necessary since the extractor 200 can be forced over the lead.

When hard tissue, e.g., plaque, is encountered so that the extractor 200 cannot be further advanced sufficiently easy over the lead, the user actuates trigger 340 (Figure 42) to thereby pull inner cable 333 proximally, which pulls the carrier 240 proximally since cable 331 is attached to the fixed proximal ring 242. When the carrier 240 is pulled back, shown by the proximally pointing arrows of Figure 26, the extractor 200 is advanced distally over the lead A as proximal clamping ring 260 clamps lead A. Note the more relative movement of carrier 240 and lead A, the more tilting of the proximal clamping ring 260 and more clamping force applied to the lead A. Simultaneous with such proximal movement of the carrier, the outer housing 202 rotates, preferably about 45 degrees although other degrees of rotation are also contemplated, due to the engagement of tabs 276a, 276b (of upper support 274) and the engagement of tabs 282a, 282b (of lower support 280) with the internal helical slot 218 of outer housing 202. This axial and rotational movement of outer housing 202, in cooperation with the stationary (non-rotating) cutting portion 224 of inner housing 204, facilitates the cutting portions cutting through tissue around the lead A. This retracted position of carrier 240 is also shown in Figure 31. Note as the carrier 240 is retracted, distal clamp ring 290 is pivoted about upper hinge point in a clockwise direction, compressing spring 302. The relative movement of the extractor 200 and lead A causes the distal clamping ring 290 to move to this less angled position of Figure 26 to facilitate movement of the extractor 200 as the opening in the distal clamping ring 290 provides a larger diameter with respect to the outer diameter of the lead A and no longer provides a restrictive clamping force on the lead A. Although the angle of the proximal clamping member 260 might not substantially change during such retraction of carrier 240, remaining in substantially the same position as in Figure 25, biased by spring 266, it will tilt more if needed such as if a larger force is applied.

Next, the trigger 252 is returned to the neutral position (Figure 41), thereby pushing cable 331 distally, which pushes the carrier 240 distally in the direction of the arrow of Figure 27 to reset the extractor 200 for the next incremental movement. As shown in Figures 27 and 31, in the initial movement of the carrier 240 distally, the interaction with the lead A pivots the proximal clamping ring 260 about its bottom hinge in a counterclockwise direction to a more vertical position, thereby compressing spring 266, and creating a larger diameter gap about opening 264 with respect to the outer diameter of the lead A to facilitate movement of the extractor 200 with respect to the lead A. The carrier 240 thereby moves to the distal position of Figures 28 and 33, with proximal clamping ring 260 remaining in the less tilted (and unclamped) position as a result of such movement. Distal clamping member 290 returns to the tilted position of Figure 25 as the extractor 200 is moved relative to lead A and it remains in the tilted clamping position to clamp the lead A and prevent the lead shifting back, i.e., reversing itself. Such distal movement of carrier 240 causes the outer housing 202 to rotate during its axial advancement due to the tab/helical slot 218 engagement discussed above, the axial movement and rotation of the cutter (rotating relative to the fixed cutting portion of inner housing 204) cutting tissue around the lead A. Note the clamping member 290 prevents the lead from moving back and there is no (or little) relative movement of the lead and extractor 200. However, the outer housing 202 will still rotate upon movement of the carrier 240, thus making the same but opposite cutting movement when the carrier 240 is moved distally.

After full distal travel of the carrier 240 with respect to the lead A, the carrier 240 returns to the position of Figures 29 and 34, which is the same position of Figures 25 and 30. Note that the relative movement of the extractor 200 and the lead A causes automatic tilting of the clamping rings 260 and 290. That is, due to the angular positioning of the clamping rings 290, 260, and the top and bottom hinge points, they operate as follows: when the carrier 240 is moved proximally to swallow the lead A, proximal clamping ring 290 remains in the same angular position (although it is moved axially) and distal clamping ring 290 is rotated by the lead to a less angled position; and when the carrier 240 is moved distally to reset, the distal clamping ring 290 returns, due to the lead (and assisted by spring 302), to the tilted (angular) position to prevent reverse relative movement with the lead and the proximal clamping ring 260 is tilted by the lead A to a less angled (move vertical) position. Such rotation or tilting of proximal clamping ring 260 compresses the biasing spring 266.

Note that the proximal and distal clamping rings 260, 290 do not perform a clamping function when they are not sufficiently tilted, i.e., when they are in a substantially vertical position. The springs 266, 302 aid the clamping rings 260, 290 in making the initial tilting to a more angled position. As soon as the clamping rings 260, 290 start locking on the lead as a result of relative axial movement, the tilting increases and the locking force increases. The greater the force, the better the locking on the lead.

The above steps of Figures 25-29 are then repeated the desired number of times by actuation of the trigger (actuating member) 340. As can be appreciated, the trigger or actuator 340 is repeatedly pulled and released, to cause progressive and incremental relative movement of the extractor 200 and lead A to cut, e.g., sever and/or dissect, tissue adjacent the lead and "swallow" the lead A to free the lead from the surrounding tissue so it can be removed from the body. As can also be appreciated, this movement and tunneling action of the extractor 200 results in the removal force applied at the distal end of the device, adjacent the tissue engagement of the lead.

In certain instances it may be desirable to quickly abort the procedure and quickly remove the extractor 200 from the lead. This requires the clamping rings 260, 290 to be moved to the less tilted unclamping position. This is shown in Figures 43 and 44. Figures 41 and 42 show normal use of the trigger 352. As noted above, when trigger 340 is pulled back, it pulls back on cable 331 to retract the carrier 240; when trigger 340 is moved forward (distally), it pushes the carrier 240. This is the normal use of trigger 252 to achieve desired movement of the carrier and relative movement ("swallowing") of the lead. However, if during the procedure, the user desires to quickly remove the extractor 200, the trigger 340 is moved to its forwardmost (distalmost) position to move the carrier 240 to an advanced override position. This override position is distal of the carrier position of Figures 25 and 30. In this position, the carrier 240 is advanced so the distal edge 275 of upper support 274 contacts a proximal end 290a of distal clamping ring 290 forcing it to a less tilted position, and in some embodiments a positon close to about 90 degrees with respect to the longitudinal axis of the lead A. Such movement of the carrier 240 with respect to the lead A also causes the proximal clamping ring 260 to move to the less tilted position as it does in Figures 27 and 28. Thus, with the less tilted position and larger gaps of the openings 292 and 264 in clamping rings 290, 260 with respect to the outer diameter of the lead, the extractor 200 can more freely slide over the lead A and be removed from the patient's body. Note a detent can be provided to limit movement of the trigger to the position of Figure 41, and then overridden by application of sufficient force to move the trigger to the override position. Alternatively, a latch or other locking mechanism can be provided to restrict movement of the trigger to the position of Figure 41, and released to allow movement of trigger 340 to the position of Figure 43 to cancel the procedure. Also, a retention mechanism can be provided to retain the trigger in the override position.

Note in some embodiments the trigger 340 can be in the neutral position of Figure 41 and then return to the position of Figure 41 when released from the position of Figure 42. Also, the trigger mechanism can include a stop such as a detent, which would prevent movement of the trigger 340 to the override position of Figure 43 during its normal use, and require sufficient force of the trigger 340 to override the detent to force it into the override position of Figure 43.

Note alternatively an external power source such as a motor can be provided to electrically drive (actuate) the cable 333 instead of manual operation by the user.

In an alternate embodiment not forming part of the present invention illustrated in Figures 37-40, a flexible sheath 370 is provided which enables unscrewing of the lead at the distal end. The sheath 370 also has sufficient rigidity to allow for rotation. The lead extractor 200' is the same as lead extractor 200 except for the provision of the sheath positioned over a portion of the extractor 200'. The extractor 200' is used in the identical fashion as extractor 200 described above to separate the lead from the tissue encapsulating the lead along its length. Therefore, for brevity, the components of the extractor 200' and their function will not be repeated herein as they are identical to extractor 200, and identical components, e.g., outer housing 202', distal clamping ring 290', and proximal clamping ring 260' are labeled with "prime" designations. The sheath 370 is attached to the proximal end cap 249' of the inner tube 204' and extends proximally of the end cap 249', forming an extension of the inner tube 204'. After the extractor 200' has completed its tunneling action as described above and the lead A is free from tissue proximal to its embedded distal end, the flexible sheath 370 is rotated (Figure 38), which in turn rotates the extractor 200'. Since the lead A is firmly clamped by the extractor 200', rotation of the sheath 370 also rotates the lead A, thereby unscrewing the distal tip B of the lead A which is embedded in tissue (Figure 39). The sheath 370, extractor 200' and clamped lead A can then be removed from the body as shown in Figure 40.

Figures 45-56 illustrate an embodiment of a lead extractor of the present invention. The lead extractor is designated generally by reference numeral 400. The lead extractor 400 is similar to lead extractor 200 described above in that it is configured to incrementally move relative to the lead to "swallow" the lead in increments i.e., the extractor 400 advances relative to the fixed lead, the lead retracts relative to the extractor 400, or both the extractor 400 and lead simultaneously move in distal and proximal directions to effect extraction of the lead. However, in the lead extractor 400, instead of the clamping members in the form of rings having openings to surround the lead, a plurality of discrete clamping members, in the form of jaws or "claws", are spaced apart circumferentially to engage spaced regions around the circumference of the lead. This enables use of the lead extractor 400 with different sized leads. It also limits blocking of the lead extractor by debris (pollution) as it can ride over the debris if needed. Other differences between extractor 400 and extractor 200 will become apparent from the detailed description below of the lead extractor 400.

Turning to the components of lead extractor 400, and with initial reference to Figures 45 and 46, lead extractor 400 has an outer body 402 (also referred to herein as an outer tube or outer housing) having a proximal portion 412 and a distal portion 408. A cutter or cutting portion 406 is formed at the distal portion 408 and preferably includes a serrated edge or toothed edge to effectively cut tissue adjacent the lead. Outer body 402 is positioned coaxially over inner housing (or inner tube) 416 as the inner housing 416 is received in lumen 410 of outer body 402. Outer body 402 has an internal helical slot similar to helical slot 218 of outer body 202 of lead extractor 200 discussed above, and is rotatable relative to inner housing 416 in the same manner as discussed above to cut (sever and/or dissect) tissue in the same manner as outer body 202 as the structures described below are moved axially to effect extraction of the lead. Outer housing 402 is preferably cylindrically shaped with a conical tip 402a at the distal portion 408 tapering in a distal direction to facilitate tunneling of the lead extracting device 400 through tissue. Radial slots 415 receive the two semi-circular discs 420 attached to the inner housing 416 and are welded to the outer tube 402 to help attach the outer body 402 and inner housing 416. Also, the disc 420 blocks axial movement of the outer body 402 so that when the carrier 430 moves axially as described below, axial movement of the outer body 402 is blocked, and it moves only in a rotational movement. In one embodiment, the outer housing 402 rotates about 60 degrees, although other degrees of rotation are also contemplated.

The inner housing 416 has a cutter or cutting portion 417 at a distal end, preferably having a serrated or toothed edge as shown, which interacts with the cutting portion 406 of outer housing 402 to sever (cut or dissect) tissue adjacent the lead. That is, the cutting portion 406 of outer housing 402 overlies a counterpart cutting portion 417 of inner housing 416. A circumferential slot 419 is formed in inner housing 416 to receive semi-circular discs (plates) 420.

The inner housing 416 includes a distal clamping structure and a proximal clamping structure. Distal clamping structure includes a stationary distal jaw support 418 and the proximal clamping structure includes a proximal movable carrier (vehicle) 430. The carrier 430 is supported by inner housing 416, positioned over the tubular portion 427 which extends proximally from jaw support 418. The carrier 430 is mounted proximal of the stationary jaw support 418 and is movable axially between proximal and distal positions to effect extraction or "swallowing" the lead as described in detail below. The carrier 430 slides within the three elongated axial slots 426 of tubular portion 427. Contained with the lumen 428 of the inner housing 416 is elongated override tube 442, also discussed in detail below.

The jaw support 418 is connected to the inner housing 416 and is positioned distally of the movable carrier 430. Jaw support 418 supports three clamping members or jaws 466 which function as claws to frictionally grasp the lead extending through lumen 428 of inner housing 416. Thus, jaw support 418 provides a stationary distal clamping structure. By stationary, the distal clamping structure 418 is not movable proximally by the movable carrier 430 and also prevents return of the lead and/or extractor 400 as discussed below. The distal jaw support 418 allows relative movement of the lead and extractor 400 in an extraction direction, but prevents relative movement or at least substantial relative movement of the lead and extractor 400 in the reverse direction.

As shown, the three jaws 466 (also referred to herein as clamps or clamping members) are spaced apart about 120 degrees to provide gripping of the lead at spaced apart regions, e.g., about 120 degrees apart around the circumference of the lead. It should be appreciated that although three jaws are shown, a different number of spaced apart jaws could also be provided and/or at a different spacing. Each jaw 466 is mounted within a distal radial (circumferential) slot 422 of stationary jaw support 418. Each jaw 466 has a series of teeth 472 angled in a direction that enables relative movement of the lead and extractor 400 in an extraction direction when frictionally engaged thereto, but prohibits relative movement of the lead and extractor 400 in the reverse direction when so engaged. An opening 467 in each of the jaws 466 receives distal jaw ring 462. That is, the jaws 466 are mounted on ring 462 in a key-ring fashion. Distal jaw ring 462 has three regions 474 which are each fitted in one of the distal radial slots 422. Three T-shaped springs 468 are provided - each spring 468 biasing an associated jaw 466 toward the lead, i.e. toward the longitudinal axis of the lead extractor/device 400. The springs 468 are shown mounted to a ring 464 in a key-ring fashion. The springs 468 can each include an opening (not shown) similar to opening 467 for passage of the ring 464. Ring 464 has three regions for mounting within the three proximal radial (circumferential) slots 424 of the distal jaw support 418. Radial slots 424 are positioned proximally of radial slots 422. Although the springs 468 are shown mounted on a ring 464, in alternate embodiments, a ring is not provided and the springs are individually mounted within the jaw support 418. The transverse portions 469 of the T-shaped springs 468 are seated within the radial slots 424 and the longitudinal portions are seated within the longitudinal slots 425 of the jaw support 418 which extend axially to connect the radial slots 422, 424.

Turning now to details of the movable carrier 430 which provides a proximal clamping structure and with continued reference to Figure 46, the movable carrier 430 has a helical rib 432 on an external surface engageable with the internal helical slot of the outer housing 402. In this manner, when the carrier 430 is moved axially, it causes rotation of the outer housing 402 to rotate the cutter 406. In one embodiment, the outer housing 402 is rotated about 60 degrees in one direction when the movable carrier 430 is moved (retracted) its full distance and then rotated in the opposite direction back to its original position when the movable carrier 430 is moved back to its initial (distal) position. It should be appreciated that the movable carrier 430 and housing 402 are relatively movable in that movement of the carrier 430 in a proximal direction causes relative movement the housing 402 in the opposite direction.

The movable carrier 430, also referred to herein as the proximal jaw support, supports three jaws 486 (also referred to herein as clamping members or clamps) which function as claws to frictionally grasp the lead extending through lumen 428 of inner housing 416. However, unlike distal jaw support 418, movable carrier 430 is designed to move back and forth axially to effect swallowing the lead. When pulled back, the force applied by the teeth 492 of the jaws 486 mounted in the movable carrier 430 maintain their clamping force on the lead so that the lead and extractor 400 can be relatively moved as the movable carrier 430 is pulled proximally, e.g., the outer housing 402 advanced distally along the lead. When the movable carrier 430 is moved distally back to its original distal position, the force applied by the jaws 486 to the lead is overridden so the movable carrier 430 can be slid distally over the lead, with the teeth of distal jaws 466 maintaining a sufficient clamping force on the lead to prevent relative movement, or at least substantial relative movement, of the lead and extractor, so any movement does not return the extractor 400 or lead to its previous position, as the movable carrier 430 is passed over the lead. Cable 461, contained within cable sheath 460, effects movement of the movable carrier 430 as described below.

As shown, the jaws 486 are spaced apart about 120 degrees to provide gripping of the lead at spaced apart regions, e.g., about 120 degrees apart around the circumference. It should be appreciated that although three jaws are shown, a different number of spaced apart jaws could also be provided and/or a different spacing. Each jaw 486 is mounted within a distal radial (circumferential) slot 434 of movable carrier 430. Each jaw 486 has a series of teeth 492 frictionally engaging the lead to apply a sufficient gripping force to effect relative movement of the lead when it is pulled proximally, e.g. by the cable 461 discussed below. An opening 490 in each of the jaws 486 receives proximal jaw ring 482. That is, the jaws 486 are mounted on ring 482 in a key-ring fashion. Proximal jaw ring 482 has three regions which are each fitted in one of the distal radial slots 434 of movable carrier 430. Three T-shaped springs 488 are provided - each spring 488 biasing an associated jaw 486 toward the lead, i.e., toward the longitudinal axis of the device 400. The springs 488 are shown mounted to a spring ring 484 in a key-ring fashion. The springs 488 can each include an opening (not shown) similar to opening 490 for passage of ring 484. Spring ring 484 also has three regions for mounting within the three proximal radial (circumferential) ring slots 436 of the movable carrier 430. Radial slots 436 are positioned proximally of radial slots 434. Although the springs 488 are shown mounted on a ring 484, in alternate embodiments, a ring is not provided and the springs are individually mounted within the movable carrier 430. As shown, the transverse portions 489 of the T-shaped springs 488 are seated within the radial slots 436 and the longitudinal portions are seated within the longitudinal slots 440 of the movable carrier 430 which extend axially to connect radial slots 434 and 436.

Various mechanisms can be utilized to retract and advance the movable carrier 430. In the illustrated embodiment, by way of example, a cable 461 contained within a sheath 460, is attached within an opening 435 in the proximal end of the movable carrier 430 and welded to the carrier 430 therein. As shown, the cable sheath 460 and inner cable 461 extend through opening 458 in proximal end ring 456. The cable 461 can be actuated by various mechanisms (actuators) accessible outside the patient. For example, in one embodiment, illustrated in Figure 56, a proximal portion of the cable 461 is connected to a lever 470. Lever 470 is preferably biased to an open position corresponding to a distal position of the movable carrier 430. When the lever 470 is squeezed, it pulls the cable 461 proximally which pushes the cable 461 to move the attached movable carrier 430 proximally. When the lever 470 is released, it moves the carrier 430 distally to return it to its original distal position. As can be appreciated, although the lever 470 is configured so that actuation pulls the movable carrier 430 proximally, it can alternatively be configured so that actuation pushes the movable carrier 430 distally and release returns the movable carrier to its original proximal position. The actuator is shown in the form of a trigger-like lever, however, it should be appreciated that other types of actuators, e.g. sliding levers, are also contemplated.

An override tube 442 (Figure 46) is positioned within lumen 428 of inner tube 416 and functions to release the lead extractor 400 from the lead if desired during the procedure. Override tube 442 has three axially extending distal slots 444 and three axially extending proximal slots 446. Lumen 450 of override tube 442 is configured to allow passage of the lead therethrough. Slots 446 accommodate movement of the movable carrier 430. A distal edge 447 of each slot 444 is configured to contact one of the distal jaws 466 to disengage the jaws 466 from frictional engagement with the lead. A distal edge 448 of each slot 446 is configured to contact one of the proximal jaws 486 to disengage the jaws 486 from frictional engagement with the lead. Thus, during the procedure, if the user desires to disengage the device 400 from the lead to remove it from the lead, the override tube 442 is pulled proximally so that edges 447, 448 engage the respective jaws 466, 486 and force the jaws 466, 486 upwardly away from the lead, i.e., away from the longitudinal axis of the device 400 against the bias or respective springs 486, 488 to thereby release the frictional clamping of the lead so the device 400 can be slid over the lead and removed.

Cable 455 contained within cable sheath 454 is attached within a proximal opening 452 of override tube 442 and welded therein. Proximal movement of the cable 455 thereby retracts the override tube 442. Various mechanisms can be utilized to retract the override tube 442. For example, as shown in Figure 56, a lever 480, accessible outside the patient, can be operably connected to a proximal end of the cable 455. Lever 480 is preferably biased to an open position corresponding to a distal position of the override tube 442. When the lever 480 is squeezed, it pulls the cable 455 proximally which moves the attached override tube 442 proximally. When the lever 480 is released, it moves the override tube 442 distally to return to its original distal position. Other actuators are also contemplated such as slidable levers to effect movement of the override tube 442.

Use of the extractor 400 will now be described. The extractor 400 is first inserted over a proximal end of the lead, e.g., a cardiac lead, which is embedded in tissue and desired to be removed. The extractor 400 is advanced until the distal end 408 of the outer housing 402 encounters hard tissue. Note, in the insertion position, the movable carrier 430 (proximal jaw support) is in the distal position adjacent the distal jaw support 418 as shown in Figures 47, 48, and 53. The extractor 400 can be forced distally over the lead A overriding the frictional force of jaws 466 and 486 on the lead A. Note in this position, override tube 442 is in the distal position. Note in Figures 47, 48, and 53 (and 49-52), the outer housing 402 is not shown in order to show the internal components. It should also be appreciated, that in alternate embodiments, the extractor 400 can be inserted with the movable carrier 430 in the proximal position so the proximal position is the initial position.

When hard tissue, e.g., plaque, is encountered so that the extractor 400 cannot be further advanced sufficiently easy over the lead, the user actuates the cable actuator such as trigger (lever) 472 of Figure 56 to thereby pull inner cable 461 proximally, which pulls the movable carrier 430 proximally since cable 461 is attached to the movable carrier 430. When the carrier 430 is pulled back, shown by the proximally pointing arrows of Figure 49 and 50, relative movement of the carrier 400 and lead A occurs as the lead A is frictionally engaged by the clamping force of jaws 486. This relative movement of carrier 430 "swallows" the lead. Simultaneous with such proximal movement of the carrier 430 and/or distal movement of the housing 402 (relative movement of the carrier 430 and housing 402), the outer housing 402 rotates, preferably about 60 degrees although other degrees of rotation are also contemplated, due to the engagement of helical rib 432 of carrier 430 with the internal helical slot of the outer housing 402. This rotational movement of outer housing 402, in cooperation with the stationary (non-rotating) cutting portion 417 of inner housing 416, facilitates the cutting portions cutting through tissue around the lead A. Note as the carrier 430 is retracted, distal jaw support 418 is not retracted. The proximal position of the carrier 430 is illustrated in Figures 49 and 50.

Next, the lever 470 is returned to the neutral (original) position of Figure 56, thereby pushing cable 461 distally, which pushes the movable carrier 430 distally in the direction of the arrows of Figure 51 and 52 to reset the extractor 400 for the next incremental movement. The movable carrier 430 thereby moves distally until it returns to the distal position of Figures 47 and 48, with the force applied by the cable 461 on the carrier 430 overriding the clamping force of the jaws 486 so the movable carrier 430 can be slid distally back to its original distal position of Figure 47. Distal jaw support 418 remains stationary (or substantially stationary) with jaws 466 applying a sufficient clamping force to prevent relative movement of the lead A and extractor 400 as the movable carrier 430 is moved distally. This prevents the lead A or extractor 400 shifting back, i.e., reversing itself. Such distal movement of the movable carrier 430 causes the outer housing 402 to rotate back in the reverse direction due to the helical rib 432 engagement discussed above. Note the axial distance of travel of the movable carrier 430 is defined by the length of the slot 446 of the override tube 442, i.e. distal edge 448 providing a distal stop and proximal edge 449 providing a proximal stop. Axial movement of the outer housing 402 is blocked by disc (plates) 420. That is, the outer housing 402 is blocked from axial movement but still rotates upon return of the movable carrier 430 to its initial position, thus making the same but opposite cutting movement when the carrier 430 is moved distally.

After full distal travel of the movable carrier 430 with respect to the lead A, back to its original position of Figures 47 and 48, the user once again actuates lever 470 to pull cable 461 and movable carrier 430 proximally, to further extract the lead A, i.e., further swallow the lead due to the relative movement. That is, the above steps of Figures 47-52 are then repeated the desired number of times by actuation of the trigger (actuating member) 470. As can be appreciated, the actuator 470 is repeatedly pulled and released, to cause progressive and incremental removal of the lead by the extractor 400 to cut, e.g., sever and/or dissect, tissue adjacent the lead and "swallow" the lead A to free the lead from the surrounding tissue so it can be removed from the body. As can also be appreciated, this movement and tunneling action of the extractor 400 results in the removal force applied at the distal end of the device, adjacent the tissue engagement of the lead.

In certain instances it may be desirable to quickly abort the procedure and quickly remove the extractor 400 from the lead. This requires the clamping jaws 466 and 486 to be disengaged from frictional engagement with the lead. That is, if during the procedure, the user desires to quickly remove the extractor 400, the trigger 480 of Figure 56 is actuated, i.e., squeezed, to retract the cable 455 which causes retraction of the attached override tube 442 to an override position. This override position of the override tube 442 is proximal of its initial (distal) position shown in Figure 54. As override tube 442 is retracted, edges 447 of slots 444 apply a force to the respective distal jaw 466 and edges 448 of slots 446 apply a force to proximal jaws 486 against the force of the springs 468, 488. The edges 447, 448 thereby lift the jaws 468, 488 away from the longitudinal axis of the device 400 so the teeth 472, 492 no longer frictionally engage and restrain the lead A, as shown in Figure 55. Thus, with the jaws 466 and 486 disengaged, the extractor 400 can more freely slide over the lead A and be removed from the patient's body. Note a retention mechanism can be provided to retain the actuator 480, e.g., trigger 480, in the override position.

Note in an alternate embodiment an external power source such as a motor can be provided to electrically drive (actuate) the cable 461 and/or cable 455 instead of manual operation by the user.

In an alternate embodiment a flexible sheath can provided which enables unscrewing of the lead at the distal end in the same manner as flexible sheath 370 of Figures 37-40 described above. The sheath is positioned over a portion of the extractor 400, and the extractor 400 is used in the identical fashion as extractor 400 described above to separate the lead from the tissue encapsulating the lead along its length. The sheath can be attached to the proximal end cap 456 of the extractor 400 and extends proximally of the end cap 456, forming an extension. After the extractor 400 has completed its tunneling action as described above and the lead A is free from tissue proximal to its embedded distal end, the flexible sheath can be rotated in the same manner as in Figure 38, which in turn rotates the attached extractor 400. Since the lead A is firmly clamped by the extractor 400, rotation of the sheath also rotates the lead A, thereby unscrewing the distal tip of the lead A which is embedded in tissue. The sheath, extractor 400 and clamped lead A can then be removed from the body.

Although described for extracting a lead, the extractors of the present disclosure can also be utilized in other surgical applications.

While specific embodiments have been described above, it will be appreciated that the invention may be practiced otherwise than as described. Moreover, specific items discussed with reference to any of the isolated drawings may freely be inter-changed supplementing each other in any particular way. The descriptions above are intended to be illustrative, not limiting. Thus, it will be apparent to one skilled in the art that modifications may be made to the invention as described in the foregoing without departing from the scope of the claims set out below. The present invention is set out in the appended claims. The embodiments, examples or aspects according to the present description which do not fall within the scope of said claims are provided for illustrative purposes only and do not form part of the present invention.

## Claims

1. An extractor for removing an implanted lead (A) from a patient, the extractor comprising
a lumen (410) dimensioned to receive the lead therein;
a cutter (406) at a distal portion (408) for cutting tissue adjacent the lead;
a distal clamping structure (418) spaced proximally of the cutter; and
a proximal clamping structure (430) spaced proximally of the distal clamping structure, the proximal clamping structure relatively movable with respect to the distal clamping structure to extract the lead from the patient,
**characterized in that** the proximal clamping structure includes a first plurality of discrete radially spaced apart clamping members (486).

2. An extractor as claimed in claim 1, wherein each of the discrete clamping members (486) has a series of teeth (492) adapted to frictionally engage the lead (A) to apply a sufficient gripping force to effect relative movement of the lead when it is pulled proximally.

3. An extractor as claimed in claim 1 or 2, further comprising a movement mechanism (432) operatively associated with the proximal clamping structure (430), the movement mechanism movable between first and second positions to move the proximal clamping structure between distal and proximal positions.

4. An extractor as claimed in any preceding claim, wherein movement of the proximal clamping structure (430) proximally effects extraction of the lead (A) and movement of the proximal clamping structure distally resets the proximal clamping structure for subsequent movement of the proximal clamping structure for further extraction of the lead.

5. An extractor as claimed in any preceding claim, wherein the proximal clamping structure (430) comprises a first plurality of springs (488), the first plurality of springs biasing the first plurality of clamping members (486) in a direction toward a longitudinal axis of the extractor.

6. An extractor as claimed in any preceding claim, wherein the distal clamping structure (418) is substantially stationary and enables relative movement of the lead (A) in response to proximal movement of the proximal clamping structure (430) but prevents relative movement of the lead in a reverse direction.

7. An extractor as claimed in claim 6, wherein the distal clamping structure (418) includes a second plurality of discrete radially spaced apart clamping members (466).

8. An extractor as claimed in claim 7, further comprising a second plurality of springs (468), the second plurality of springs biasing the second plurality of clamping members (466) in a direction toward a longitudinal axis of the extractor.

9. An extractor as claimed in any preceding claim, further comprising a cable (461) operatively associated with the proximal clamping structure (430), wherein proximal movement of the cable retracts the proximal clamping structure proximally to effect extraction of the lead (A).

10. An extractor as claimed in claim 9, wherein distal movement of the cable (461) resets the proximal clamping structure (430) for subsequent proximal movement to further extract the lead (A).

11. An extractor as claimed in any preceding claim, wherein the cutter (406) is rotatable concurrently with axial movement of the proximal clamping structure (430).

12. An extractor as claimed in any preceding claim, further comprising a flexible sheath (460), the flexible sheath rotatable with the extractor to unscrew a distal tip of the lead (A) from tissue.

13. An extractor as claimed in any preceding claim, further comprising an override mechanism (442) to release the proximal and distal clamping structures (430, 418) to enable removal of the extractor from the lead (A).

14. An extractor as claimed in claim 13, wherein the distal clamping structure (418) includes a second plurality of radially spaced apart clamping members (466), and the override mechanism (442) includes a slidable member engageable with the first and second plurality of clamping members (486, 466) to force the first and second plurality of clamping members out of frictional engagement with the lead (A) against the force of a plurality of springs (488, 468).

15. An extractor as claimed in any preceding claim, wherein the extractor and lead (A) are incrementally relatively movable to swallow the lead as tissue is cut by the cutter (406) adjacent the lead.

## Patentansprüche

1. Extraktor für die Entfernung einer implantierten Leitung (A) aus einem Patienten, wobei der Extraktor Folgendes umfasst:
ein Lumen (410), das zur Aufnahme der Leitung darin dimensioniert ist;
ein Schneidelement (406) an einem distalen Teil (408) zum Schneiden von Gewebe, das an die Leitung angrenzt;
eine distale Klemmstruktur (418), die proximal von dem Schneidelement beabstandet ist; und
eine proximale Klemmstruktur (430), die proximal von der distalen Klemmstruktur beabstandet ist, wobei die proximale Klemmstruktur relativ in Bezug auf die distale Klemmstruktur beweglich ist, um die Leitung aus dem Patienten zu extrahieren,
**dadurch gekennzeichnet, dass** die proximale Klemmstruktur eine erste Vielzahl von separaten, radial beabstandeten Klemmelementen (486) einschließt.

2. Extraktor nach Anspruch 1, wobei jedes der separaten Klemmelemente (486) eine Reihe von Zähnen (492) aufweist, die zum reibschlüssigen Eingreifen in die Leitung (A) geeignet sind, um eine ausreichende Greifkraft anzuwenden, um die relative Bewegung der Leitung zu bewirken, wenn sie proximal gezogen wird.

3. Extraktor nach Anspruch 1 oder 2, der weiter einen Bewegungsmechanismus (432) umfasst, der funktionsfähig mit der proximalen Klemmstruktur (430) verbunden ist, wobei der Bewegungsmechanismus zwischen ersten und zweiten Positionen beweglich ist, um die proximale Klemmstruktur zwischen distalen und proximalen Positionen zu bewegen.

4. Extraktor nach einem vorstehenden Anspruch, wobei die Bewegung der proximalen Klemmstruktur (430) proximal die Extraktion der Leitung (A) bewirkt und die Bewegung der proximalen Klemmstruktur distal die proximale Klemmstruktur für die anschließende Bewegung der proximalen Klemmstruktur für die weitere Extraktion der Leitung zurücksetzt.

5. Extraktor nach einem vorstehenden Anspruch, wobei die proximale Klemmstruktur (430) eine erste Vielzahl von Federn (488) umfasst, wobei die erste Vielzahl von Federn die erste Vielzahl von Klemmelementen (486) in einer Richtung zu einer Längsachse des Extraktors vorspannt.

6. Extraktor nach einem vorstehenden Anspruch, wobei die distale Klemmstruktur (418) im Wesentlichen stationär ist und die relative Bewegung der Leitung (A) als Reaktion auf die proximale Bewegung der proximalen Klemmstruktur (430) ermöglicht, doch die relative Bewegung der Leitung in einer umgekehrten Richtung verhindert.

7. Extraktor nach Anspruch 6, wobei die distale Klemmstruktur (418) eine zweite Vielzahl von separaten, radial beabstandeten Klemmelementen (466) einschließt.

8. Extraktor nach Anspruch 7, der weiter eine zweite Vielzahl von Federn (468) umfasst, wobei die zweite Vielzahl von Federn die zweite Vielzahl von Klemmelementen (466) in einer Richtung zu einer Längsachse des Extraktors vorspannt.

9. Extraktor nach einem vorstehenden Anspruch, der weiter ein Kabel (461) umfasst, das funktionsfähig mit der proximalen Klemmstruktur (430) verbunden ist, wobei die proximale Bewegung des Kabels die proximale Klemmstruktur proximal zurückzieht, um die Extraktion der Leitung (A) zu bewirken.

10. Extraktor nach Anspruch 9, wobei die distale Bewegung des Kabels (461) die proximale Klemmstruktur (430) für die anschließende proximale Bewegung zurücksetzt, um die Leitung (A) weiter zu extrahieren.

11. Extraktor nach einem vorstehenden Anspruch, wobei das Schneidelement (406) gleichzeitig mit der axialen Bewegung der proximalen Klemmstruktur (430) drehbar ist.

12. Extraktor nach einem vorstehenden Anspruch, der weiter eine flexible Hülse (460) umfasst, wobei die flexible Hülse mit dem Extraktor drehbar ist, um eine distale Spitze der Leitung (A) aus dem Gewebe herauszudrehen.

13. Extraktor nach einem vorstehenden Anspruch, der weiter einen Aufhebungsmechanismus (442) umfasst, um die proximale und distale Klemmstruktur (430, 418) freizugeben, um die Entfernung des Extraktors von der Leitung (A) zu ermöglichen.

14. Extraktor nach Anspruch 13, wobei die distale Klemmstruktur (418) eine zweite Vielzahl von radial beabstandeten Klemmelementen (466) einschließt und der Aufhebungsmechanismus (442) ein verschiebbares Element einschließt, das in die erste und zweite Vielzahl von Klemmelementen (486, 466) eingreifen kann, um die erste und zweite Vielzahl von Klemmelementen aus dem reibschlüssigen Eingriff mit der Leitung (A) gegen die Kraft einer Vielzahl von Federn (488, 468) zu drängen.

15. Extraktor nach einem vorstehenden Anspruch, wobei der Extraktor und die Leitung (A) stufenweise relativ beweglich sind, um die Leitung aufzunehmen, während das Gewebe durch das Schneidelement (406) angrenzend an die Leitung geschnitten wird.

## Revendications

1. Extracteur pour retirer une sonde implantée (A) d'un patient, l'extracteur comprenant
une lumière (410) dimensionnée pour recevoir la sonde en son sein ;
un dispositif de coupe (406) au niveau d'une partie distale (408) destiné à couper un tissu adjacent à la sonde ;
une structure de serrage distale (418) espacée proximalement du dispositif de coupe ; et
une structure de serrage proximale (430) espacée proximalement de la structure de serrage distale, la structure de serrage distale étant relativement mobile par rapport à la structure de serrage distale pour extraire la sonde du patient,
**caractérisé en ce que** la structure de serrage proximale comporte une première pluralité d'éléments de serrage radialement espacés discrets (486).

2. Extracteur selon la revendication 1, dans lequel chacun des éléments de serrage discrets (486) présente une série de dents (492) adaptée pour venir en contact par frottement avec la sonde (A) en vue d'appliquer une force de saisie suffisante pour effectuer un déplacement relatif de la sonde lorsqu'elle est tirée proximalement.

3. Extracteur selon la revendication 1 ou 2, comprenant en outre un mécanisme de déplacement (432) associé fonctionnellement à la structure de serrage proximale (430), le mécanisme de déplacement étant mobile entre des première et seconde positions pour déplacer la structure de serrage proximale entre des positions distale et proximale.

4. Extracteur selon n'importe quelle revendication précédente, dans lequel un déplacement de la structure de serrage proximale (430) effectue proximalement une extraction de la sonde (A) et un déplacement de la structure de serrage proximale rétablit distalement la structure de serrage proximale en vue d'un déplacement ultérieur de la structure de serrage proximale pour extraire davantage la sonde.

5. Extracteur selon n'importe quelle revendication précédente, dans lequel la structure de serrage proximale (430) comprend une première pluralité de ressorts (488), la première pluralité de ressorts sollicitant la première pluralité d'éléments de serrage (486) dans une direction vers un axe longitudinal de l'extracteur.

6. Extracteur selon n'importe quelle revendication précédente, dans lequel la structure de serrage distale (418) est sensiblement fixe et permet un déplacement relatif de la sonde (A) en réponse à un déplacement proximal de la structure de serrage proximale (430) mais empêche un déplacement relatif de la sonde dans un sens inverse.

7. Extracteur selon la revendication 6, dans lequel la structure de serrage distale (418) comprend une seconde pluralité d'éléments de serrage radialement espacés discrets (466).

8. Extracteur selon la revendication 7, comprenant en outre une seconde pluralité de ressorts (468), la seconde pluralité de ressorts sollicitant la seconde pluralité d'éléments de serrage (466) dans une direction vers un axe longitudinal de l'extracteur.

9. Extracteur selon n'importe quelle revendication précédente, comprenant en outre un câble (461) associé opérationnellement à la structure de serrage proximale (430), dans lequel un déplacement proximal du câble rétracte la structure de serrage proximale proximalement pour effectuer une extraction de la sonde (A).

10. Extracteur selon la revendication 9, dans lequel un déplacement distal du câble (461) rétablit la structure de serrage proximale (430) en vue d'un déplacement proximal suivant pour extraire davantage la sonde (A).

11. Extracteur selon n'importe quelle revendication précédente, dans lequel le dispositif de coupe (406) est rotatif de manière concomitante au déplacement axial de la structure de serrage proximale (430).

12. Extracteur selon n'importe quelle revendication précédente, comprenant en outre une gaine souple (460), la gaine souple pouvant tourner avec l'extracteur pour dévisser une pointe distale de la sonde (A) du tissu.

13. Extracteur selon n'importe quelle revendication précédente, comprenant en outre un mécanisme d'annulation (442) pour libérer les structures de serrage proximale et distale (430, 418) afin de permettre le retrait de l'extracteur de la sonde (A).

14. Extracteur selon la revendication 13, dans lequel la structure de serrage distale (418) comporte une seconde pluralité d'éléments de serrage radialement espacés (466), et le mécanisme d'annulation (442) comporte un élément coulissant pouvant venir en contact avec les première et seconde pluralités d'éléments de serrage (486, 466) pour forcer les première et seconde pluralités d'éléments de serrage hors de leur contact par frottement avec la sonde (A) en opposition à la force d'une pluralité de ressorts (488, 468).

15. Extracteur selon n'importe quelle revendication précédente, dans lequel l'extracteur et la sonde (A) peuvent être déplacés d'une manière relativement incrémentielle pour avaler la sonde au fur et à mesure que le tissu est coupé par le dispositif de coupe (406) adjacent à la sonde.
